# EUROPEAN PATENT SPECIFICATION

(11) **EP 3 945 256 B1**
(45) Date of publication and mention of the grant of the patent: **21.05.2025**
(21) Application number: 21187765.9
(22) Date of filing: 26.07.2021
(51) Int. Cl.: F24F 8/108, F24F 8/22, F24F 13/20, F24F 13/28, F24F 7/003, F24F 7/007, F24F 8/80, B01D 46/00, B01D 46/10

(54) **PORTABLE AIR CARING APPARATUS**
TRAGBARES LUFTPFLEGEGERÄT
APPAREIL PORTABLE DE SOIN D'AIR

(30) Priority: 27.07.2020 KR 20200093401; 11.12.2020 KR 20200173594
(43) Date of publication of application: 02.02.2022
(73) Proprietor: LG Electronics Inc., Seoul 07336 (KR)
(72) Inventor: KIM, Juhyun, Seoul (KR); OH, Si Young, Seoul (KR); KIM, Kidong, Seoul (KR); CHOI, Seok-Ho, Seoul (KR)
(74) Representative: Vossius & Partner Patentanwälte Rechtsanwälte mbB

(56) References cited:
- CN-A- 105 509 181
- CN-A- 109 812 891
- JP-A- 2002 143 282
- KR-A- 20190 029 478
- KR-B1- 102 132 859
- US-A1- 2010 089 243

## Description

### BACKGROUND

### 1. Field of the Invention

The present invention relates to a portable air caring apparatus, and more particularly, to a portable air caring apparatus capable of sanitizing a filter portion.

### 2. Discussion of Related Art

An air caring apparatus is an apparatus widely used in modern life that purifies air by filtering physical particles such as dust, fine dust, and ultrafine dust, chemical substances such as odorous particles and harmful gases, and microorganisms such as bacteria and viruses.

Due to the influence of urbanization, industrialization, and globalization, the air caring apparatus has become an indispensable apparatus also in general homes. Also, the demand for the air caring apparatus has also sharply increased due to the influence of an increase in fine dust levels, an increase in the number of allergy patients, an improvement in the standard of living, and the like.

The air caring apparatus may have a large size when targeting an environment larger than 100 m², such as a general home. In the apparatus, a filter that handles physical particles such as dust, a filter that handles chemical substances such as gases, and a filter that handles microorganisms bacteria and viruses may be used in combination. That is, in a large space, an air caring apparatus of a large size that can accommodate various filters together may be used.

However, using an air caring apparatus of a large size in a small space such as a studio apartment or the inside of a vehicle, an extremely large space such as a public library, or outdoors is inefficient in terms of space utilization, mobility, and energy consumption. Also, for users who often move from place to place, an air caring apparatus that has a small size and can be easily carried for use by an individual is more suitable than an air caring apparatus having a large size. Under such a background, a portable air caring apparatus that can be easily carried for use by an individual is being developed.

The portable air caring apparatus is provided in a small, lightweight form so that it is easy to carry. The portable air caring apparatus has an advantage in that it can easily be carried and used at a desired location by a user. That is, the portable air caring apparatus is an apparatus suitable for users who tend to frequently go out or move from place to place rather than staying in one place, such as their home, for a long period of time.

A related art (Korean Patent Publication No. 10-2019-0029478 A, Title of Invention: Portable air caring apparatus) is an invention relating to an air caring apparatus in which a rear panel configured to suction air and a front panel configured to discharge air are installed in a horizontal direction. Therefore, outside air that passes through the rear panel and enters a case sequentially passes through a filter portion and a fan assembly and then is discharged to the outside of the portable air caring apparatus through a discharge port. However, the related art does not include a separate sanitizing apparatus for sanitizing the filter portion, and thus there is a problem in that the filter portion is prone to contamination. Also, when the separate sanitizing apparatus for sanitizing the filter portion is installed inside the portable air caring apparatus of the related art, a separate fixing bracket for fixing the sanitizing apparatus should be additionally installed inside the case. Since the separate fixing bracket is additionally installed inside the case, the sizes of the filter portion and the fan assembly become relatively small, and thus there is a problem in that an air caring ability is degraded. Also, when the additionally installed sanitizing apparatus is fixed to a fan module portion in the portable air caring apparatus of the related art, the sanitizing light irradiated from the sanitizing apparatus interferes with a filter case that fixes the filter portion, and thus there is a problem in that sanitization of the filter portion is not performed properly. Also, when the sanitizing apparatus is installed at the fan module portion, there is a problem in that frictional resistance between the sanitizing apparatus and air suctioned through an inlet of the fan module portion increases and thus an air blowing ability is degraded. In addition, the case of the portable air caring apparatus of the related art is formed in a rectangular parallelepiped shape extending in a vertical direction, and corners of the case are provided at an outer side of the case. Therefore, an inconvenience may occur when a user holds the outer side of the case, and when mounting the portable air caring apparatus on a structure such as a cup holder having the shape of a groove which is concave toward the lower side, there is a problem in that a gap is formed between the case and the cylindrical groove-shaped cup holder and the gap causes the case to shake, which becomes a cause of vibration and noise.

KR 102 132 859 B1 relates to a portable air purifier having a filter sterilization function, and a portable air purifier having a filter sterilization function capable of increasing the life of the filter by continuously sterilizing the filter

### SUMMARY OF THE INVENTION

The invention is defined by independent claim 1. Further embodiments of the invention are defined by the dependent claims. The present invention is directed to providing a portable air caring apparatus capable of preventing contamination of air by installing a separate sanitizing apparatus for sanitizing a filter portion.

The present invention is also directed to providing a portable air caring apparatus capable of, in a case in which a sanitizing apparatus is installed inside the portable air caring apparatus, minimizing an increase in resistance of an air flow path passing through the portable air caring apparatus to achieve high air caring performance.

The present invention is also directed to providing a portable air caring apparatus capable of, in a case in which the portable air caring apparatus is installed in a cup holder, reducing an occurrence of vibration and noise of the portable air caring apparatus.

The present invention is also directed to providing a portable air caring apparatus allowing a user to conveniently hold an outer side of a case and carry the case.

Objectives of the present invention are not limited to the above-mentioned objectives, and other unmentioned objectives of the present invention and advantages thereof should be understood from the following description and should be more clearly understood from embodiments of the present invention. Also, it should be easily understood that the objectives and advantages of the present invention may be realized by means shown in the claims below and combinations thereof.

To achieve the above objectives, a portable air caring apparatus according to the present invention has a sanitizing portion installed between a filter portion and a fan module portion to sanitize the filter portion.

Specifically, the sanitizing portion is mounted on the filter case and configured to irradiate sanitizing light toward the filter portion so that the filter portion is sanitized by sanitizing light irradiated from the sanitizing portion. Also, since the sanitizing portion is installed between the fan module portion and the filter portion, and the sanitizing light is irradiated toward the filter portion which blocks an inlet portion, the sanitizing light which is harmful to the human body may be prevented from being exposed to the outside of a housing portion.

Also, in the portable air caring apparatus according to the present invention, since the sanitizing portion is installed in a shape extending in a vertical direction from the center of the fan module portion, an increase in resistance of an air flow path may be minimized.

Specifically, in a state in which a plurality of fan members are continuously installed in the vertical direction, since an upper end and a lower end of the sanitizing portion extending in the vertical direction do not protrude to the outside of a hub of the fan member, interference of the sanitizing portion with an air flow path along which air is suctioned into the fan members may be minimized.

Also, in the portable air caring apparatus according to the present invention, in a state in which an air flow path passing through the housing portion in a horizontal direction is formed, the sanitizing portion may be installed between the filter portion and the fan module portion.

Specifically, since air entering the inlet portion of the housing portion sequentially passes through the filter portion, the sanitizing portion, and the fan module portion while moving in the horizontal direction and then is discharged through an outlet portion of the housing portion, a movement path of the air is short, and thus resistance of an air flow path may be reduced.

Also, in the portable air caring apparatus according to the present invention, with respect to a first horizontal center line that passes through the center of the housing portion in the horizontal direction and is located at the center of the inlet portion and the outlet portion, a distance between the filter portion and the fan module portion may vary.

Specifically, for the sanitizing portion to irradiate the sanitizing light toward the filter portion, the sanitizing portion and the filter portion should be spaced apart from each other at a predetermined gap, and since an air blowing ability of the fan module portion is an important factor in an air caring function, the fan module portion may be installed to be closer to the first horizontal center line than the filter portion. Also, the volume of the fan module portion may be larger than the volume of the filter portion.

Also, in the portable air caring apparatus according to the present invention, a transverse cross-section of the housing portion that is installed in a concave groove portion, such as a cup holder of a vehicle, may be formed in a circular shape or an elliptical shape.

Specifically, since the transverse cross-section of the housing portion is formed in the circular or elliptical shape, the housing portion may be stably seated on the groove portion of the cup holder having a circular transverse cross-section, and a separation distance between the housing portion and the cup holder may be reduced such that the possibility of an occurrence of vibration and noise is reduced.

Also, in the portable air caring apparatus according to the present invention, the sanitizing portion may be fixed between the fan module portion and the filter case.

Specifically, since a front surface of the sanitizing portion is supported by a fixing protrusion provided on the filter case, and a rear surface of the sanitizing portion is supported by the fan module portion, additionally installing a separate fixing bracket for fixing the sanitizing portion is not necessary, and thus an increase in resistance of an air flow path may be minimized.

Also, in the portable air caring apparatus according to the present invention, a protruding rib may be provided on an outer side of a bell mouth in a radial direction, and the protruding rib may be spaced apart from a shroud at a predetermined gap and have an inclined surface that is parallel to an inclined surface of the shroud. Thus, an occurrence of flow loss due to return air may be suppressed, and since rigidity of a fan base is increased, deformation of the fan base due to an external force may be suppressed.

The portable air caring apparatus according to the present invention may include the housing portion, the filter portion, the fan module portion, the filter case, and the sanitizing portion.

The housing portion may have an accommodation space formed therein and may include the inlet portion configured to suction air and the outlet portion configured to discharge air. Also, the housing portion may include a first case portion and a second case portion.

The filter portion, the sanitizing portion, and the fan module portion may be installed inside the first case portion. Also, the inlet portion may be provided at one side surface of the first case portion, and the outlet portion configured to discharge air may be provided at the other side surface of the first case portion that faces the inlet portion.

Also, the first case portion may include a first case body and a service door. The first case body may include the inlet portion and the outlet portion and have a service space provided at a side surface.

The service door may be detachably installed at the first case body so as to open and close the service space. Also, the service door may include a door body, a gap maintaining protrusion, and a first mounting groove portion. Also, the door body may be detachably installed at the first case body. Also, the gap maintaining protrusion may protrude to the inside of the door body. Also, the gap maintaining protrusion may extend toward at least any one of the filter portion and the filter case.

Also, the first mounting groove portion may form a groove inside the door body in the vertical direction.

Also, the first case body may further include a second mounting groove portion configured to form a groove at an inner side of the first case body, which faces the first mounting groove portion, in the vertical direction. Also, one side and the other side of the fan module portion in a width direction may be inserted into the first mounting groove portion and the second mounting groove portion to restrict movement of the fan module portion.

Also, in the inlet portion, a plurality of inlet grilles extending in the vertical direction may be installed at one side surface of the housing portion so as to form inlet holes. Also, the inlet grilles may extend in a longitudinal direction and guide the movement path of air in the longitudinal direction. Also, in the outlet portion, a plurality of outlet grilles extending in the vertical direction may be installed at the other side surface of the housing portion so as to form outlet holes. Also, the outlet grilles may extend in the longitudinal direction and guide the movement path of air in the longitudinal direction. Also, the inlet holes and the outlet holes may face each other in the horizontal direction. Also, a length of the inlet portion in the width direction may be shorter than a length of the filter portion in the width direction.

Also, the second case portion may be connected to a lower portion of the first case portion. Also, a battery may be embedded in the second case portion.

An air flow path may be formed inside the first case portion, and an air flow path may not be formed inside the second case portion. Also, a transverse cross-section of at least any one of the first case portion and the second case portion may be formed in a circular shape or an elliptical shape. Also, the first case portion and the second case portion may be separately formed and then assembled together or may be integrally formed.

Also, the filter portion may be installed inside the housing portion facing the inlet portion. Also, the filter portion may be disposed inside the housing portion and installed in a shape that faces the inlet portion. Also, the filter portion may be installed in a state in which both sides thereof in the width direction come in contact with the inside of the housing portion. Also, the filter portion has a rectangular parallelepiped shape extending in the vertical direction. Also, the filter portion may be mounted inside the filter case such that movement thereof is restricted.

Also, the filter portion may be detachably installed in the filter case, and the filter case may be disposed between the filter portion and the fan module portion. Also, the filter case may guide movement of air from the filter portion to the fan module portion.

Also, the filter case may include at least any one of a case main body portion, a flow path guiding portion, a fixing supporting portion, a catching step, a first fixing protrusion, a second fixing protrusion, and a first coupling protrusion.

The case main body portion may be fixed to the inside of the housing portion, and the filter portion may be mounted inside the case main body portion. Also, both longitudinal sides of the case main body portion may be open.

Also, the flow path guiding portion may be installed at one side of the case main body portion and may form a path along which air moves from the filter portion toward the fan module portion.

Also, the fixing supporting portion may be disposed at the center of the flow path guiding portion. Also, the fixing supporting portion may extend in the vertical direction and may include a protrusion configured to support the sanitizing portion.

Also, the catching step may protrude to the inside of the case main body portion and restrict movement of the filter portion. Also, the filter portion caught on the catching step may be installed to be spaced a predetermined distance apart from the sanitizing portion.

Also, the first fixing protrusion may protrude to the upper side of the case main body portion and be fixed to a first boss disposed at an inner side of the housing portion. Also, the second fixing protrusion may protrude to the lower side of the case main body portion and be fixed to a second boss disposed at an inner side of the housing portion.

The first coupling protrusion may protrude in a direction toward the fan module portion and be inserted into the fan module portion.

Also, the flow path guiding portion may be installed at both sides of the fixing supporting portion, extend in the vertical direction, and include a plurality of through-holes.

Also, the fixing supporting portion may include a filter frame, which is configured to form an inner hole that extends in the vertical direction and connected to the flow path guiding portion, and a plurality of fixing protrusions that protrude from the filter frame toward the inner hole.

Also, the sanitizing portion may be mounted on the filter case and irradiate the sanitizing light toward the filter portion. Also, the sanitizing portion may be inserted into the filter frame, and movement of the sanitizing portion may be restricted due to the sanitizing portion being caught on the fixing protrusion. Also, the sanitizing portion may be installed at the center of the filter case in the width direction and may extend in the vertical direction. Also, the sanitizing portion may include a printed circuit board which extends in the vertical direction and a sanitizing light source which is installed at both sides of the printed circuit board in the vertical direction and is configured to irradiate the sanitizing light toward the filter portion. Also, the sanitizing portion may be disposed at the center of the fan module portion in the width direction. Also, two fan members may be installed in the vertical direction along the sanitizing portion. Also, an upper end of the sanitizing portion may be installed at a position lower than a position of an upper end of a hub of the fan member that is disposed at the upper side of the sanitizing portion. Also, a lower end of the sanitizing portion may be installed at a position higher than a position of a lower end of the hub of the fan member that is disposed at the lower side of the sanitizing portion. Also, an irradiation angle of the sanitizing light irradiated by the sanitizing light source may be in a range of 140° to 160°.

Also, the fan module portion may be installed inside the housing portion facing the outlet portion. Also, the fan module portion may include a fan housing, the fan member, and the fan base. The fan housing may be fixed to an inner side of the housing portion. The fan member may be rotatably installed inside the fan housing and may move air in a direction toward the outlet portion. Also, the fan base may be coupled to the fan housing and may guide the air, which has passed through the filter portion, to enter the fan member. Also, a length of the fan module portion in the width direction may be formed to be longer than a length of a discharge portion in the width direction.

Also, the fan housing may be coupled to the fan base while surrounding the fan member. Also, the fan housing, the fan member, and the fan base may constitute a module. Also, as the fan housing and the fan member, two fan housings and two fan members may be installed in the vertical direction of the fan base. Also, a central axis of rotation of the fan member may intersect the sanitizing portion at a right angle.

Also, the fan housing may include at least any one of a support plate, a connection support, a side support portion, and a protruding boss. The support plate may be installed in the shape of a plate at a position facing the fan member. The connection support may extend toward the outside of the support plate. Also, the side support portion may be connected to the connection support and may form a circular or quadrilateral frame around the periphery of the support plate. Also, the protruding boss may protrude in a direction from the side support portion toward the fan base and may be coupled to the fan base.

Also, the fan housing may include a wire guide which is continuously installed on the connection support and configured to support a wire installed along a side surface of the connection support. Also, the fan housing may include a wire fixing protrusion that protrudes from the side support portion and is disposed in a lateral direction of the wire, which is disposed at the outer side of the connection support, to prevent detachment of the wire.

Also, the fan member may include a hub that is disposed at the center of the fan housing and configured to receive external power and rotate. Also, the fan member may include a plurality of fan blades disposed to be spaced apart from each other at equal intervals along an outer peripheral surface of the hub. Also, the fan member may include a shroud that is connected to an end portion of the fan blade, installed in an annular shape, and spaced apart from the fan base.

Also, an outer diameter of the hub and an inner diameter of the shroud may gradually decrease in a direction from the outlet portion toward the inlet portion.

Also, the fan base may include at least any one of a base plate, a bell mouth, a coupling protrusion, and a protruding rib.

The base plate is formed in the shape of a plate that includes a hollow for air movement and extends in the vertical direction. Also, the bell mouth may be installed in an annular shape at an inner side of the base plate that faces the hollow. Also, the bell mouth may include a longitudinal end surface that has a concave shape surrounding a lower side of an inlet protrusion of the shroud. Also, the coupling protrusion may protrude from the base plate and be coupled to the protruding boss of the fan housing. Also, the protruding rib may be installed in an annular shape surrounding the outer periphery of the bell mouth. Also, the protruding rib may be installed to be parallel to the outer side of the fan member.

Also, air that enters through the inlet portion may be guided in the horizontal direction so that the air sequentially passes through the filter portion, the filter case, and the fan module portion and then is discharged to the outside of the housing portion through the outlet portion.

Also, the first horizontal center line passing through the center of the housing portion in the transverse direction may be disposed at the center of the inlet portion and the outlet portion.

Also, when a distance between the first horizontal center line and the filter portion is D1 and a distance between the first horizontal center line and the fan module portion is D2, D1 and D2 may have different values. Also, D1 may have a greater value than D2.

Also, a second horizontal center line that intersects the first horizontal center line at a right angle at the center of the housing portion and extends in the transverse direction may pass through the center of the inlet portion, the filter portion, the sanitizing portion, the fan module portion, and the outlet portion in the width direction.

Also, as the fan member disposed in the fan module portion, a plurality of fan members may be installed in the vertical direction. Also, the sanitizing portion may be installed at a position parallel to a vertical virtual line that perpendicularly intersects the central axis of rotation of the fan member and extends in the vertical direction. Also, one side surface of the sanitizing portion may be supported by the fixing protrusion of the filter case. Also, the other side surface of the sanitizing portion may be supported by the fan module portion.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a perspective view illustrating a portable air caring apparatus according to an embodiment of the present invention;
FIG. 2 is a front view of the portable air caring apparatus according to an embodiment of the present invention;
FIG. 3 is a rear view of the portable air caring apparatus according to an embodiment of the present invention;
FIG. 4 is a plan view of the portable air caring apparatus according to an embodiment of the present invention;
FIG. 5 is a perspective view illustrating a state in which a second case portion is separated from the portable air caring apparatus according to an embodiment of the present invention;
FIGS. 6 and 7 are exploded perspective views of the portable air caring apparatus according to an embodiment of the present invention;
FIG. 8 is a view illustrating an air flow path passing through the portable air caring apparatus according to an embodiment of the present invention in a horizontal direction;
FIG. 9 is an exploded perspective view of a housing portion according to an embodiment of the present invention;
FIG. 10 is a plan cross-sectional view of the portable air caring apparatus according to an embodiment of the present invention;
FIG. 11 is a view illustrating a movement direction of air passing through the portable air caring apparatus according to an embodiment of the present invention;
FIG. 12 is a front view illustrating a state in which a sanitizing portion is installed between a fan module portion and a filter portion according to an embodiment of the present invention;
FIGS. 13 and 14 are perspective views illustrating a state in which a filter case is disposed between the sanitizing portion and the filter portion according to an embodiment of the present invention;
FIG. 15 is a perspective view illustrating the filter case according to an embodiment of the present invention;
FIG. 16 is a view illustrating a state in which the sanitizing portion is installed at the center of the fan module portion according to an embodiment of the present invention;
FIG. 17 is a perspective view illustrating a state in which a fan housing is separated from the fan module portion according to an embodiment of the present invention;
FIG. 18 is a perspective view illustrating the fan housing according to an embodiment of the present invention;
FIG. 19 is a perspective view illustrating the inside of the fan housing according to an embodiment of the present invention;
FIG. 20 is a perspective view illustrating a shroud of a fan member according to an embodiment of the present invention;
FIG. 21 is a perspective view illustrating an axial coupling portion of the fan member according to an embodiment of the present invention;
FIG. 22 is a front view of the fan member according to an embodiment of the present invention;
FIG. 23 is a view illustrating a hub of the fan member according to an embodiment of the present invention;
FIG. 24 is a cross-sectional view of the fan member according to an embodiment of the present invention;
FIG. 25 is a perspective view illustrating a state in which a fan base is separated from the filter case according to an embodiment of the present invention;
FIG. 26 is a perspective view illustrating the fan base according to an embodiment of the present invention;
FIG. 27 is a view illustrating a state in which the sanitizing portion is installed in a state of being spaced apart from the filter portion according to an embodiment of the present invention;
FIG. 28 is a view illustrating a state in which sanitizing light is irradiated from the sanitizing portion toward the filter portion according to an embodiment of the present invention; and
FIG. 29 is a view illustrating a state in which a locking protrusion is installed on a rear surface of the sanitizing portion according to an embodiment of the present invention.

### DETAILED DESCRIPTION OF EXEMPLARY EMBODIMENTS

The objectives, features, and advantages will be described in detail below with reference to the accompanying drawings, and accordingly, those of ordinary skill in the art to which the present invention pertains should be able to easily practice the technical idea of the present invention. In describing the present invention, when it is determined that detailed description of a known art relating to the present invention may unnecessarily obscure the gist of the present invention, the detailed description thereof will be omitted. Hereinafter, exemplary embodiments according to the present invention will be described in detail with reference to the accompanying drawings. In the drawings, the same reference numerals are used to indicate the same or similar elements.

Although terms such as first and second are used to describe various elements, of course, the elements are not limited by the terms. The terms are only used to distinguish one element from another element, and of course, a first element may also be a second element unless otherwise stated.

Hereinafter, when an arbitrary configuration is described as being disposed on an "upper portion (or lower portion)" of an element or being disposed "above (or below)" the element, this may not only mean that the arbitrary configuration is disposed in contact with an upper surface (or lower surface) of the element but may also mean that another configuration may be interposed between the element and the arbitrary configuration disposed above (or below) the element.

Also, when a certain element is described as being "connected," "coupled," or "linked" to another element, this may mean that the element is directly connected or linked to the other element but may also mean that the element is "connected," "coupled," or "linked" to the other element via another element "interposed" therebetween or the element and the other element are "connected," "coupled," or "linked" through different elements.

Throughout the specification, each element may be provided as a single element or a plurality of elements unless particularly described otherwise.

In the specification, a singular expression includes a plural expression unless the context clearly indicates otherwise. In the application, terms such as "comprise" or "include" should not necessarily be interpreted as indicating that all of various elements or various steps described in the specification are included and should be interpreted as indicating that some of the elements or some of the steps may not be included or additional elements or steps may be further included.

Throughout the specification, "A and/or B" may refer to A, B, or A and B unless particularly described otherwise, and "C to D" may refer to C or more and D or less unless particularly described otherwise.

### [Exterior of portable air caring apparatus]

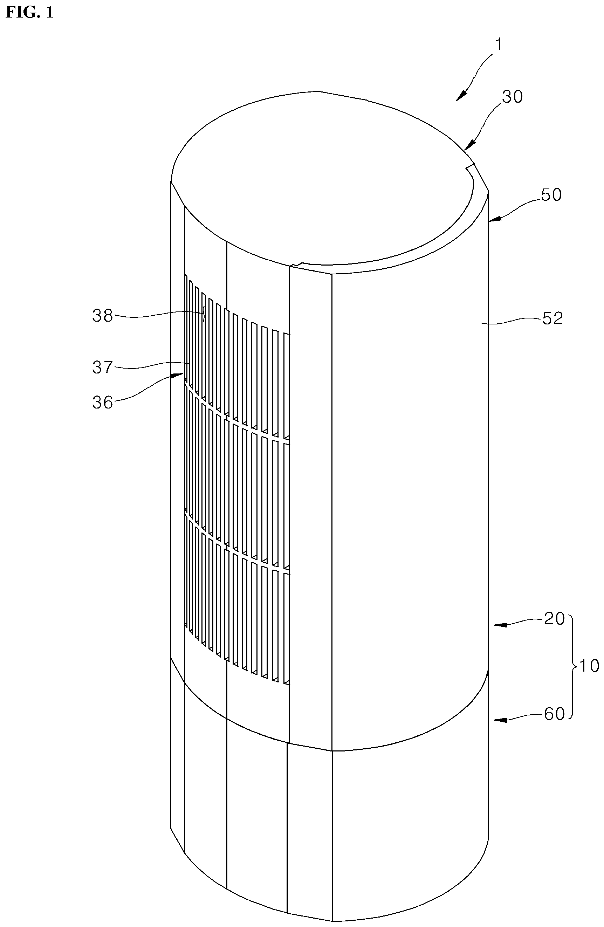

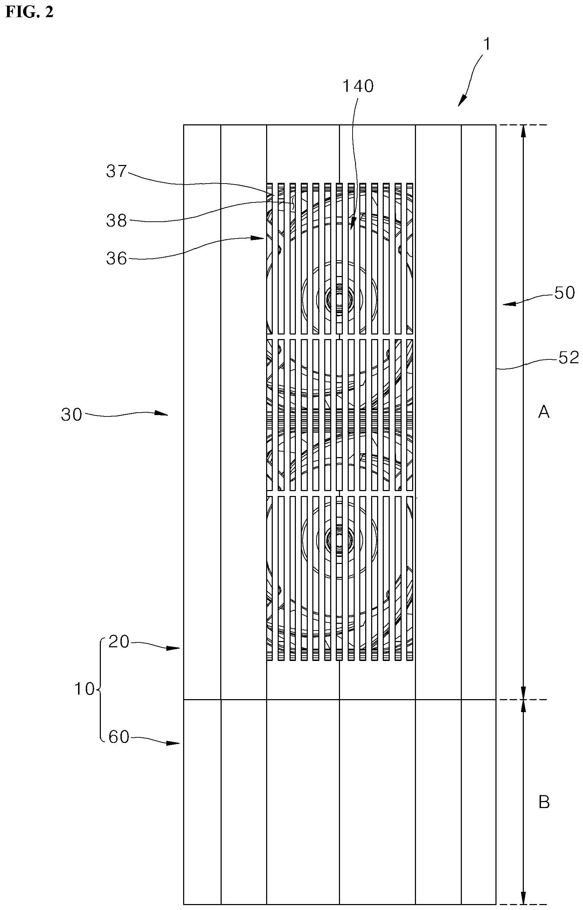

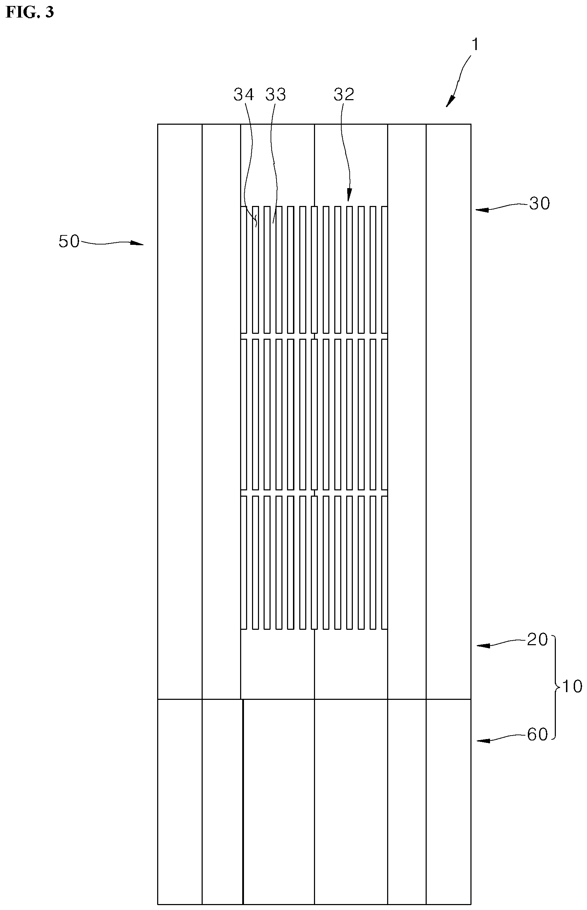

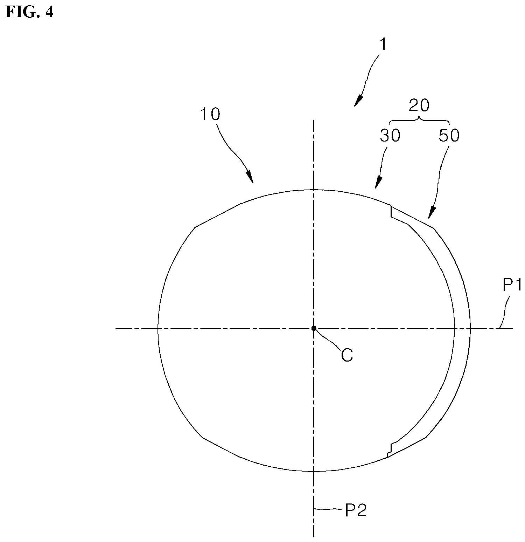

FIG. 1 is a perspective view illustrating a portable air caring apparatus 1 according to an embodiment of the present invention, FIG. 2 is a front view of the portable air caring apparatus 1 according to an embodiment of the present invention, FIG. 3 is a rear view of the portable air caring apparatus 1 according to an embodiment of the present invention, and FIG. 4 is a plan view of the portable air caring apparatus 1 according to an embodiment of the present invention.

As illustrated in FIGS. 1 to 4, the portable air caring apparatus 1 according to an embodiment of the present invention may be formed in a substantially cylindrical shape. An outer shape of the portable air caring apparatus 1 is formed by a housing portion 10. Also, the portable air caring apparatus 1 according to the present embodiment suctions air through an inlet portion 32 provided at one side surface of the housing portion 10 and discharges air through an outlet portion 36 provided at the other side surface of the housing portion 10.

The housing portion 10 may consist of a first case portion 20 and a second case portion 60. The first case portion 20 and the second case portion 60 form a framework of an exterior of the portable air caring apparatus 1. The first case portion 20 and the second case portion 60 accommodate a plurality of components.

In the first case portion 20, both sides may be formed to be open in a first direction, which is a longitudinal direction E. That is, both sides of the first case portion 20 in the longitudinal direction E may be open. The inlet portion 32 may be disposed at one side of the first case portion 20 in the first direction, and the outlet portion 36 may be disposed at the other side of the first case portion 20 in the first direction.

The inlet portion 32 and the outlet portion 36 are installed at side surfaces of the first case portion 20 that are opposite to each other. Also, since the inlet portion 32 and the outlet portion 36 are installed at positions facing each other, air moving through the inlet portion 32 is moved in the horizontal direction and discharged through the outlet portion 36. Thus, a movement path of air is shortened, and circulation of air is facilitated.

The second case portion 60 is connected to a lower side of the first case portion 20, and the second case portion 60 may be formed in a cylindrical shape. Alternatively, transverse cross-sections of the first case portion 20 and the second case portion 60 may be formed in a circular shape or an elliptical shape, and thus the first case portion 20 and the second case portion 60 may constitute the shape of a case that extends in the vertical direction.

The portable air caring apparatus 1 may be formed in the shape of a container having a curved surface on an outer side, such as a cylindrical shape or an elliptical columnar shape that stands upright and is long in the vertical direction as a whole.

Therefore, the portable air caring apparatus 1 may be inserted into a groove portion concave toward a lower side, such as a cup holder, and stably mounted. Also, since the outer side of the portable air caring apparatus 1 is bent in a curved shape, a user may easily hold the outer side of the portable air caring apparatus 1 and move the portable air caring apparatus 1.

Directions will be defined. A direction in which the first case portion 20 is located with respect to the second case portion 60 is referred to as "upper portion" or "upper side," and a direction in which the second case portion 60 is located with respect to the first case portion 20 is referred to as "lower portion" or "lower side." Also, "longitudinal direction E" and "first direction" are the same directions, and a direction in which the inlet portion 32 and the outlet portion 36 of the housing portion 10 are connected is defined as the longitudinal direction E. Also, "width direction W" and "second direction" are the same directions, and the width direction W is a direction that forms a right angle with the longitudinal direction E and extends in the horizontal direction. Also, both the longitudinal direction E and the width direction W are perpendicular to the vertical direction H. Also, "vertical direction H" and "third direction" are the same directions, and the vertical direction H and the vertical direction are the same directions. The directions are set as above on the basis of when the portable air caring apparatus 1 is coplanar with the horizontal line.

### [Overall structure of portable air caring apparatus]

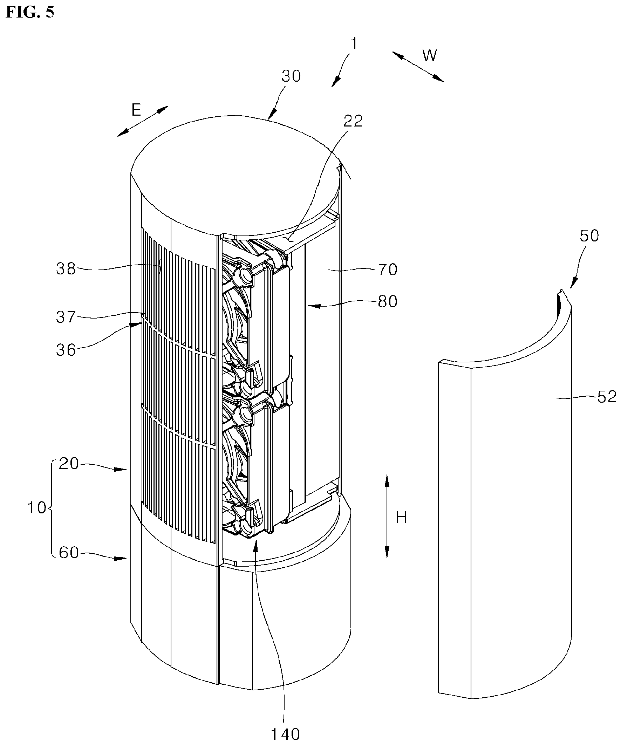

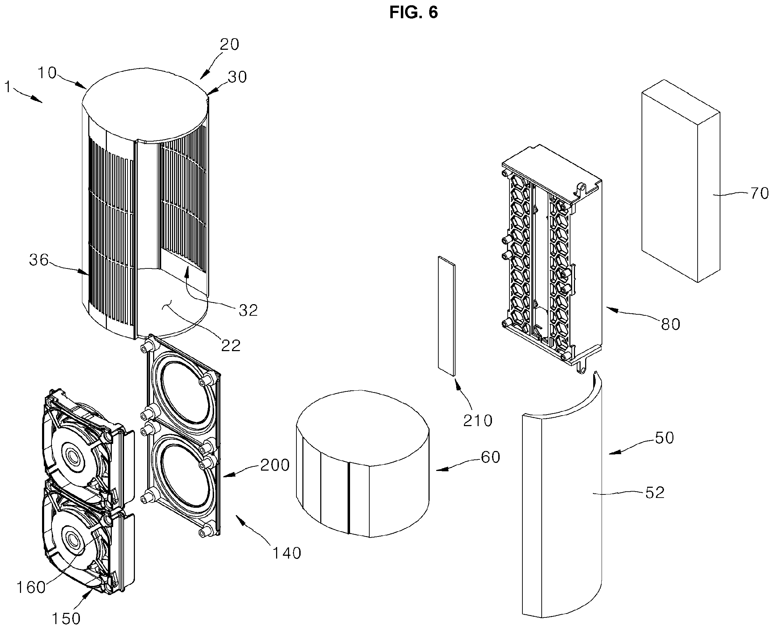

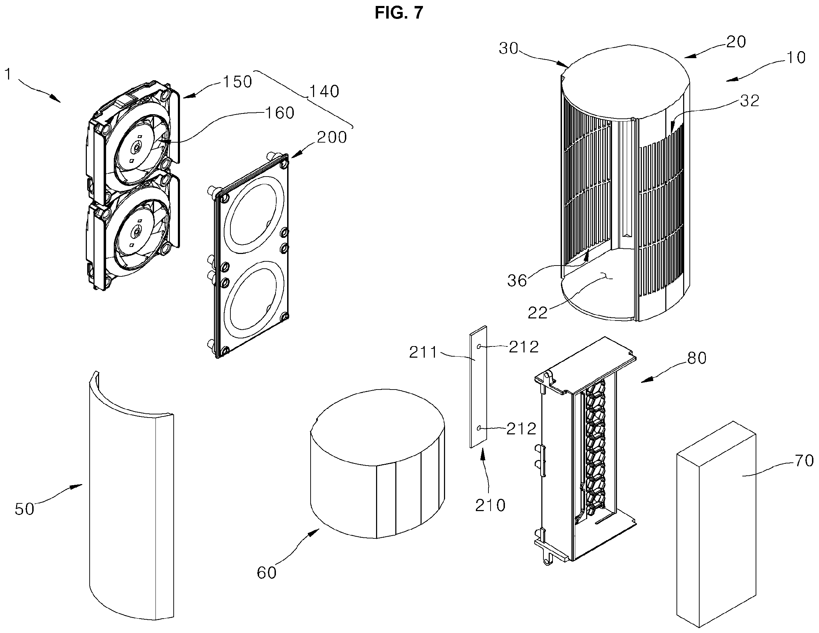

FIG. 5 is a perspective view illustrating a state in which the second case portion 60 is separated from the portable air caring apparatus 1 according to an embodiment of the present invention, and FIGS. 6 and 7 are exploded perspective views of the portable air caring apparatus 1 according to an embodiment of the present invention.

As illustrated in FIGS. 5 to 7, the portable air caring apparatus 1 according to an embodiment of the present invention may include the housing portion 10, a filter portion 70, a fan module portion 140, a filter case 80, and a sanitizing portion 210.

The first case portion 20 and the second case portion 60 form the framework of the exterior of the portable air caring apparatus 1. An accommodation space 22 is formed inside the first case portion 20 and the second case portion 60. The accommodation space 22 accommodates the filter portion 70, the filter case 80, the fan module portion 140, the sanitizing portion 210, and electronic components including a battery 220. Preferably, the first case portion 20 and the second case portion 60 are formed to have a sufficient strength to protect the accommodated components from external impact.

The filter portion 70 is installed in the accommodation space 22 of the first case portion 20 and is disposed between the fan module portion 140 and the inlet portion 32. That is, the filter portion 70 is spaced a predetermined distance apart from the fan module portion 140 and is disposed at one side of the fan module portion 140 in the longitudinal direction E. Therefore, the filter portion 70 serves to purify air that is suctioned through the inlet portion 32 of the portable air caring apparatus 1. The air purified while passing through the filter portion 70 passes through the fan module portion 140 and the outlet portion 36 and is discharged in a lateral direction of the portable air caring apparatus 1.

The filter portion 70 made be made of a single filter or, as necessary, installed in a state in which a plurality of filters are stacked. Also, the filter portion 70 is fixed by the filter case 80 for fixing the filter.

The filter case 80 is fixed to the inside of the first case portion 20, and an insertion space for accommodating the filter is formed inside the filter case 80.

The fan module portion 140 is accommodated in the accommodation space 22 inside the first case portion 20 and may be disposed between the outlet portion 36 and the filter portion 70. More specifically, the fan module portion 140 may be disposed between the outlet portion 36 and the filter case 80. Also, the sanitizing portion 210 may be installed inside the filter case 80 or installed between the filter case 80 and the fan module portion 140. In this way, various modifications are possible.

The fan module portion 140 is disposed at the other side of the filter portion 70 in the first direction, and the outlet portion 36 is disposed at the other side surface of the first case portion 20 that faces the fan module portion 140. The fan module portion 140 serves to suction air, which enters one side surface of the filter portion 70 through the inlet portion 32, and discharge the air to the other side of the first case portion 20 through the outlet portion 36 provided at the other side surface of the first case portion 20.

In the present embodiment, the fan module portion 140 is illustrated as including a diagonal flow fan. The fan module portion 140 may suction air, which has passed through the filter portion 70, in the axial direction and discharge the air in a direction between the axial direction and radial direction.

The sanitizing portion 210 is disposed between the filter portion 70 and the fan module portion 140 and may be fixed to at least any one of the filter case 80 and the fan module portion 140.

The sanitizing portion 210 may be fixed to the filter case 80. However, according to another embodiment of the present invention, the sanitizing portion 210 may be fixed to the fan module portion 140, fixed between the fan module portion 140 and the filter case 80, or fixed to the housing portion 10. In this way, various modifications are possible.

In a case in which the sanitizing portion 210 is fixed to the fan module portion 140, since a separate bracket for fixing the sanitizing portion 210 to the fan module portion 140 is necessary, production costs increase. Also, since the sanitizing portion 210 is installed in a shape that protrudes from an outer side of the fan module portion 140, the sizes of the filter case 80 and the filter portion 70 are relatively reduced, and thus air filtering performance may be degraded. Also, in a case in which the sanitizing portion 210 is installed to be integrally formed with the inside of the fan module portion 140, there is a problem in that, since the size of a fan member is reduced due to the installation of the sanitizing portion 210, an air blowing ability is degraded. Also, in a case in which the sanitizing portion 210 is installed in the fan module portion 140, there may be a problem in that the air blowing ability is degraded due to an increase in frictional resistance between the sanitizing portion 210 and the air suctioned through an inlet of the fan module portion 140.

In the present invention, in order to address the above problems, the sanitizing portion 210 is fixed to the filter case 80.

Since the sanitizing portion 210 according to an embodiment of the present invention is fixed to the filter case 80, sanitizing light irradiated by the sanitizing portion 210 is irradiated toward the filter portion 70 without interfering with the filter case 80, and thus degradation in sanitization performance of the sanitizing portion 210 may be prevented. In a case in which the sanitizing portion 210 is fixed to the fan module portion 140, since the sanitizing light irradiated by the sanitizing portion 210 interferes with the filter case 80 fixing the filter portion 70, the sanitization performance of the sanitizing portion 210 may be degraded.

Also, the filter case 80, the sanitizing portion 210, and the filter portion 70 constitute a single module and are mounted inside the housing portion 10. Since the sanitizing portion 210 sanitizes the filter portion 70 by irradiating the filter portion 70 with the sanitizing light, an installation position of the sanitizing portion 210 and a distance between the filter portion 70 and the sanitizing portion 210 are important design factors.

Therefore, in the case in which the filter case 80, the sanitizing portion 210, and the filter portion 70 are installed to constitute a single module, even when an installation position of the fan module portion 140 or the like is changed according to the model of the portable air caring apparatus 1, the distance between the sanitizing portion 210 and the filter portion 70 is a fixed design factor. Thus, sanitization of the filter portion 70 may be stably performed, and a cost increase due to changing the design may be reduced.

Also, since the sanitizing portion 210 is installed in a state of being inserted into the filter case 80, a separate bracket for fixing the sanitizing portion 210 is not necessary, and thus the production costs may be reduced. Also, since the sanitizing portion 210 is installed in the state of being inserted into the filter case 80, the size reduction of the fan module portion 140 and the filter portion 70 due to installing the sanitizing portion 210 may be prevented, and thus degradation of the air blowing ability and air filtering ability may be prevented.

Also, since the sanitizing portion 210 fixed to the filter case 80 is installed in a state of being spaced apart from the fan module portion 140, an area of the inlet through which air is suctioned into the fan module portion 140 is not decreased. However, in a case in which the sanitizing portion 210 is fixed to the fan module portion 140, the area of the inlet through which air is suctioned into the fan module portion 140 may be decreased, and thus the air blowing ability may be degraded.

Therefore, the sanitizing portion 210 may be fixed to at least any one of the filter case 80, the fan module portion 140, and the housing portion, but preferably, the sanitizing portion 210 is fixed to the filter case 80.

The sanitizing portion 210 is spaced a predetermined distance apart from the filter portion 70 and irradiates the filter portion 70 with the sanitizing light. Since the sanitizing light irradiated by the sanitizing portion 210 is harmful to the human body, the installation position of the sanitizing portion 210 is set such that the sanitizing light does not leak to the outside of the portable air caring apparatus 1 through the inlet portion 32.

Also, since the sanitizing portion 210 is installed between the filter portion 70 and the fan module portion 140 and sanitizes the filter portion 70, contamination of air may be prevented. The sanitizing portion 210 is installed between the filter case 80, which supports the filter portion 70, and the fan module portion 140, and the filter portion 70 may be sanitized by the sanitizing light irradiated by the sanitizing portion 210. Also, since the sanitizing portion 210 is installed between the fan module portion 140 and the filter portion 70, and the sanitizing light is irradiated toward the filter portion 70 which blocks the inlet portion 32, the sanitizing light which is harmful to the human body may be prevented from being exposed to the outside of the housing portion 10.

The battery 220 is installed in the accommodation space 22 provided inside the second case portion 60 and may supply power for driving the portable air caring apparatus 1.

### [Component arrangement structure of portable air caring apparatus]

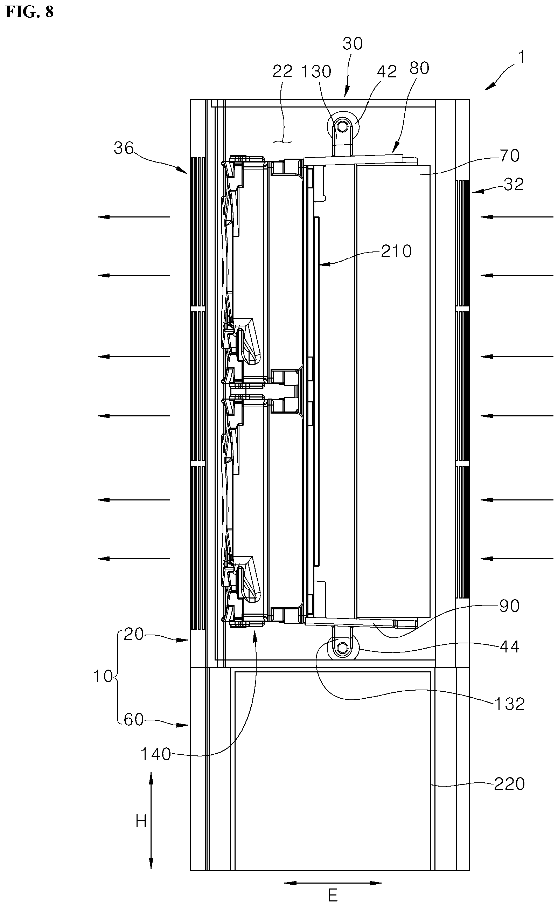

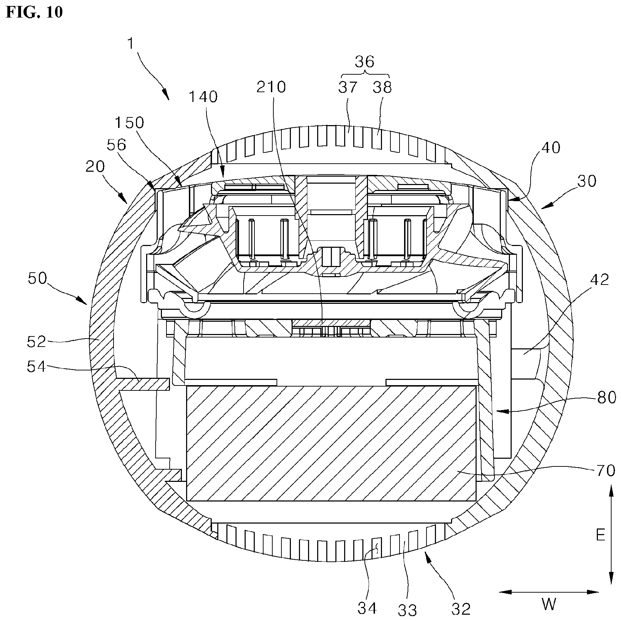

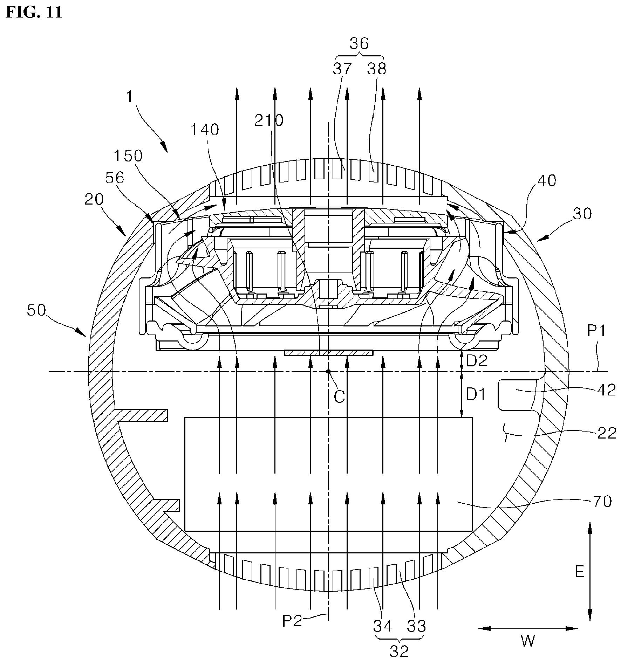

FIG. 8 is a view illustrating an air flow path passing through the portable air caring apparatus 1 according to an embodiment of the present invention in the horizontal direction, FIG. 10 is a plan cross-sectional view of the portable air caring apparatus 1 according to an embodiment of the present invention, and FIG. 11 is a view illustrating a movement direction of air passing through the portable air caring apparatus 1 according to an embodiment of the present invention.

As illustrated in FIGS. 2, 8, 10, and 11, the accommodation space 22 provided inside the portable air caring apparatus 1 may be divided into a first area A and a second area B. When the accommodation space 22 is divided in the vertical direction H, an upper area is the first area A, and a lower area is the second area B. Note that the first area A and the second area B are not physically partitioned areas and are areas that are only conceptually divided.

In an embodiment of the present invention, the accommodation space 22 of the first case portion 20 constituting the framework of the portable air caring apparatus 1 is set as the first area A, and the accommodation space 22 inside the second case portion 60 is set as the second area B.

Components relating to suctioning, purifying, and discharging air are disposed in the first area A. That is, the inlet portion 32, the filter portion 70, the fan module portion 140, and the outlet portion 36 are disposed in the first area A, and as necessary, the filter case 80 and the sanitizing portion 210 may be further installed in the first area A. Therefore, in the first area A, air flows in the horizontal direction by passing through the first case portion 20.

The inlet portion 32 having a plurality of inlet holes 34 formed therein is installed as a path for suctioning air at one side (the right side based on FIG. 8) of the first case portion 20. The outlet portion 36 having a plurality of outlet holes 38 formed therein is installed as a path for discharging the air, which is purified in the first area A, at the other side (the left side based on FIG. 8) of the first case portion 20. Therefore, an air flow path in the longitudinal direction E that connects the inlet portion 32, the filter portion 70, the fan module portion 140, and the outlet portion 36 is formed inside the first case portion 20.

That is, the inlet portion 32, the filter portion 70, the filter case 80, the sanitizing portion 210, and the outlet portion 36 are provided in the first area A, and a flow path necessary for the air, which is suctioned into the portable air caring apparatus 1, to pass through the inside of the air caring apparatus is formed in the first area A.

Components not directly related to a flow of air for purifying air are disposed in the second area B. That is, a controller, which includes a printed circuit board (PCB), and the battery 220 may be installed in the second area B.

According to the present embodiment, the first case portion 20 and the second case portion 60 are formed in a cylindrical shape or an elliptical columnar shape in which a length in the vertical direction H is longer than a length in the lateral direction. Also, the first area A disposed at an upper portion is formed to have a longer length in the vertical direction H than the second area B disposed at a lower portion. That is, when the portable air caring apparatus 1 stands upright, the first area A at the upper portion occupies a larger area than the second area B at the lower portion.

The battery 220 may be installed inside the second case portion 60 in which the second area B is formed. The battery 220 may be provided to have a heavier weight than the sum of weights of the fan module portion 140, the filter portion 70, the filter case 80, and the sanitizing portion 210.

Generally, since the weight per unit volume of the battery 220 is significantly heavier than the weight per unit volume of the fan module portion 140, the filter portion 70, the filter case 80, and the sanitizing portion 210, even when the weight or size of the battery 220 is not intentionally increased, the battery 220 has a heavier weight than the fan module portion 140, the filter portion 70, the filter case 80, and the sanitizing portion 210.

In this way, when the battery 220, which is a heavy object, is disposed in the lower portion of the portable air caring apparatus 1, the center of mass of the portable air caring apparatus 1 is biased toward the lower side from the center in the vertical direction H. That is, the center of mass of the portable air caring apparatus 1 is moved toward the lower portion of the portable air caring apparatus 1 at which the battery 220 is disposed.

In this way, when the center of mass of the portable air caring apparatus 1 is biased toward the lower side of the portable air caring apparatus 1 at which the battery 220 is disposed, a risk of the portable air caring apparatus 1 falling down when the portable air caring apparatus 1 stands upright is reduced.

That is, when the portable air caring apparatus 1 stands upright, since the center of mass of the portable air caring apparatus 1 is located at the lower side due to the battery 220 being disposed at the lower portion of the portable air caring apparatus 1, the portable air caring apparatus 1 does not fall down easily.

Also, when the battery 220, which is a heavy object, is disposed in the lower portion of the portable air caring apparatus 1, other components constituting the portable air caring apparatus 1 should be disposed above the battery 220. That is, the components relating to suctioning, purifying, and discharging air and the sanitizing portion 210 for sanitizing the filter portion 70 should be disposed at a higher position than the battery 220.

In order to secure the charge capacity of the battery 220 that is necessary for smooth use of the portable air caring apparatus 1, the size of the battery 220 is required to be a predetermined size or larger. Therefore, an installation space of a predetermined size or more for installing the battery 220 is also required inside the portable air caring apparatus 1. Also, since it is difficult to form a flow path for an air flow in the space in which the battery 220 is installed, the components relating to suctioning, purifying, and discharging air are inevitably disposed at positions avoiding the battery 220, that is, positions higher than the battery 220.

Due to such an arrangement structure, the flow path for suctioning, purifying, and discharging air is formed in the first area A, which is at a higher position than the battery 220, in the portable air caring apparatus 1. Therefore, suctioning of air into the portable air caring apparatus 1 and discharge of air purified by the portable air caring apparatus 1 are also performed at a position higher than the position where the battery 220 is installed.

When the discharge of the purified air is performed at the upper portion of the portable air caring apparatus 1 as described above, it becomes easier for the air purified by the portable air caring apparatus 1 to reach the face of a user.

When the portable air caring apparatus 1 is used while placed on a floor surface at a lower position than the user's face, in order to increase an amount of air purified by the portable air caring apparatus 1 that reaches the user's face, using the portable air caring apparatus 1 in a vertical state is more advantageous than using the portable air caring apparatus 1 in a horizontal state.

To this end, when the portable air caring apparatus 1 stands upright, the amount of air purified by the portable air caring apparatus 1 that reaches the user's face may be further increased when the discharge of the purified air is performed at the upper portion of the portable air caring apparatus 1.

Also, the structure in which the battery 220, which is a heavy object, is disposed in the lower portion of the portable air caring apparatus 1 and, accordingly, the components relating to suctioning, purifying, and discharging air are disposed at a higher position than the battery 220 may also contribute to expanding a range of installation of the portable air caring apparatus 1.

For example, when the portable air caring apparatus 1 is used while inserted into a cup holder in a vehicle, an area where suctioning of air is performed and an area where purifying and discharging of air are performed may be disposed at a higher position than the cup holder. Accordingly, air caring performance may be maintained at a high level while the portable air caring apparatus 1 is stably mounted in the vehicle. To this end, preferably, the length of the second area B in the vertical direction H in which the battery 220 is disposed may be set to be larger than or equal to a depth of the cup holder.

As another example, even in a case in which a lower area of the portable air caring apparatus 1 is fixed using a cradle in the form of tongs or the like, the portable air caring apparatus 1 can be stably fixed while an area of the portable air caring apparatus 1 where suctioning of air is performed and an area of the portable air caring apparatus 1 where discharging of the purified air is performed are not blocked.

Also, even in a case in which a user moves the portable air caring apparatus 1 while holding the lower portion of the portable air caring apparatus 1 by his or her hand, stable movement of the portable air caring apparatus 1 is possible without blocking the area of the portable air caring apparatus 1 where suctioning of air is performed and the area of the portable air caring apparatus 1 where discharging of the purified air is performed.

That is, since the components not directly related to a flow of air for purifying air are disposed at the lower portion of the portable air caring apparatus 1, and mounting and fixing of the portable air caring apparatus 1 are performed through the lower portion of the portable air caring apparatus 1, the portable air caring apparatus 1 may simultaneously provide air caring performance at a high level and be stably fixed.

In the portable air caring apparatus 1 according to an embodiment of the present invention that has the above structure, the first case portion 20 including the inlet portion 32 and the outlet portion 36 is installed at the upper side of the second case portion 60. The first case portion 20 is connected to the upper side of the second case portion 60 in which electronic components are installed, and the inlet portion 32 configured to suction air and the outlet portion 36 configured to discharge air are provided in the first case portion 20. Therefore, in a state in which the first case portion 20 is inserted into a structure such as a cup holder having the shape of a groove which is concave toward the lower side, air is easily suctioned into the first case portion 20 through the inlet portion 32 and then air is discharged in the lateral direction of the first case portion 20 through the outlet portion 36 of the first case portion 20. Thus, air cleaning efficiency may be improved.

The space inside the first case portion 20 may be divided into a direction toward the inlet portion 32 and a direction toward the outlet portion 36 with respect to a first horizontal center line P1. The first horizontal center line P1 is a virtual line passing through the center of the housing portion 10 and extends in the horizontal direction. Also, the first horizontal center line P1 is located at the center of the inlet portion 32 and the outlet portion 36.

With respect to the first horizontal center line P1, the filter portion 70 is disposed in the direction toward the inlet portion 32, and the fan module portion 140 and the sanitizing portion 210 are disposed in the direction toward the outlet portion 36. Therefore, air that has passed through the inlet portion 32 passes through the filter portion 70 such that foreign substances are removed from the air. In this state, the air moves to the inside of the fan module portion 140. Therefore, since it is possible to prevent the fan module portion 140 from being contaminated due to the foreign substances contained in the air, the durability of the fan module portion 140 may be improved, and maintenance and repair costs may also be reduced.

Also, since the sanitizing portion 210 sanitizes the filter portion 70 using the sanitizing light, an irradiation distance for irradiating the sanitizing light is essential. Therefore, in a case in which the sanitizing portion 210 is located on the first horizontal center line P1 or is installed in an area toward the inlet portion 32 with respect to the first horizontal center line P1, since an installation area of the filter portion 70 is excessively reduced, there is a problem in that air caring performance is degraded.

The area toward the inlet portion 32 with respect to the first horizontal center line P1 is an area between the first horizontal center line P1 and the inlet portion 32 as illustrated in FIG. 11.

Therefore, in a case in which the sanitizing portion 210 is installed in an area located between the first horizontal center line P1 and the outlet portion 36, an irradiation distance for irradiating the sanitizing light from the sanitizing portion 210 toward the filter portion 70 may be stably secured. Also, since it is possible to secure an installation area of the filter portion 70 for removing impurities from air entering through the inlet portion 32, degradation of air caring performance may be prevented.

However, in a case in which the sanitizing portion 210 is installed on the first horizontal center line P1 or is installed between the first horizontal center line P1 and the inlet portion 32, it is not possible to stably secure the irradiation distance for irradiating the sanitizing light from the sanitizing portion 210 toward the filter portion 70. Therefore, since sanitization of the filter portion 70 is not able to be properly performed, contaminated air may be discharged.

Also, in a case in which an installation area of the filter portion 70 is reduced to secure the irradiation distance for irradiating the sanitizing light from the sanitizing portion 210 toward the filter portion 70, air caring performance may be degraded. In order to address the above problems, the sanitizing portion 210 is installed in an area toward the outlet portion 36 with respect to the first horizontal center line P1. Thus, the installation area of the filter portion 70 is increased, and degradation of air caring performance may be prevented.

However, in a case in which a separate fixing bracket for fixing the sanitizing portion 210 is added, an installation area of the fan module portion 140 is decreased, and thus there is a problem in that air caring performance is degraded due to degradation of the air blowing ability of the fan module portion 140.

In order to address the above problem, a separate fixing bracket is not added to fix the sanitizing portion 210, and the sanitizing portion 210 is fixed between the filter case 80 and the fan module portion 140 or fixed inside the filter case 80. Thus, an increase in resistance of an air flow path may be minimized, and air caring performance may be improved.

That is, since a front surface of the sanitizing portion 210 is supported by the filter case 80 and a rear surface of the sanitizing portion 210 is supported by the fan module portion 140, it is not required to additionally install a separate fixing bracket for fixing the sanitizing portion 210, and thus an increase in resistance of an air flow path may be minimized. Also, since the installation area of the fan module portion 140 is not decreased, degradation of the air blowing ability of the fan module portion 140 may be prevented, and thus degradation of air caring performance may also be prevented.

Meanwhile, the filter portion 70 is disposed inside the inlet portion 32 and is installed in a state in which both sides of the filter portion 70 in the width direction W come in contact with the inside of the first case portion 20. Therefore, the sanitizing light irradiated toward the filter portion 70 which blocks the inlet portion 32 is prevented from leaking to the outside of the housing portion 10, and thus a safe usage environment may be provided.

### [Detailed configuration of components of portable air caring apparatus]

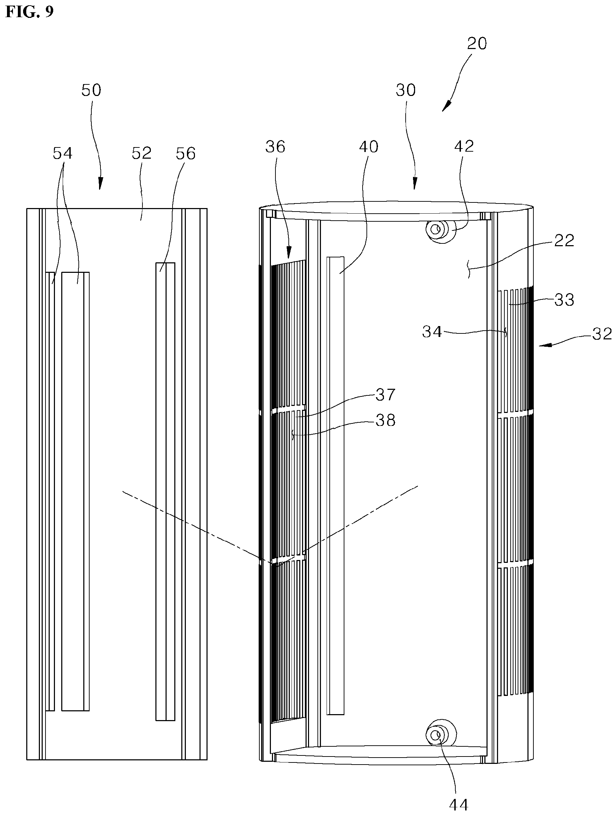

FIG. 9 is an exploded perspective view of the housing portion 10 according to an embodiment of the present invention. As illustrated in FIGS. 8 and 9, the portable air caring apparatus 1 according to an embodiment of the present invention may include at least any one of the housing portion 10, the filter portion 70, the filter case 80, the fan module portion 140, the sanitizing portion 210, and the battery 220.

The housing portion 10 has the accommodation space 22 formed therein and may be modified in various ways in which the housing portion 10 includes the inlet portion 32 configured to suction air and the outlet portion 36 configured to discharge air. The housing portion 10 according to an embodiment of the present invention may include the first case portion 20 and the second case portion 60.

The filter portion 70, the sanitizing portion 210, and the fan module portion 140 are installed inside the first case portion 20. Also, the inlet portion 32 is provided at one side surface of the first case portion 20, and the outlet portion 36 configured to discharge air is provided at the other side surface of the first case portion 20 that faces the inlet portion 32. The first case portion 20 may be made of a single member or may be made of a plurality of members as necessary. The first case portion 20 according to an embodiment of the present invention may include a first case body 30 and a service door 50.

The accommodation space 22 is formed inside the first case body 30, the inlet portion 32 configured to suction air is provided at one side surface of the first case body 30, and the outlet portion 36 is provided at the other side surface of the first case body 30 that faces the inlet portion 32.

The first case body 30 may be formed in the shape of a pipe having a cylindrical or elliptical transverse cross-section, or as necessary, the first case body 30 may have a cross-section formed in a shape in which a diameter in the width direction W is longer than a diameter in the longitudinal direction E and may extend in the vertical direction. A side surface of the first case body 30 is open to form a service space, and the service door 50 is detachably installed at the side surface of the first case body 30.

The first case body 30, which includes the inlet portion 32 and the outlet portion 36 and includes the service space formed at the side surface, may be installed in a shape in which an upper side and a lower side of the first case body 30 are blocked. The service door 50 is installed at the first case body 30 so as to be easily detachable to open and close the service space.

The first case body 30 according to an embodiment of the present invention may include at least any one of the inlet portion 32, the outlet portion 36, a second mounting groove portion 40, a first boss 42, and a second boss 44.

The inlet portion 32 includes inlet grilles 33 that extend in the vertical direction, and inlet holes 34 are provided between the plurality of inlet grilles 33. The plurality of inlet grilles 33 may be installed at one side surface of the housing portion 10 to form the inlet holes 34 for suctioning air. Since a transverse cross-section of the inlet grilles 33 extends in the longitudinal direction E, the inlet holes 34 disposed between the inlet grilles 33 are also formed in the longitudinal direction E. Therefore, movement of air that enters through the inlet portion 32 is guided in the longitudinal direction E.

Since the inlet holes 34 guide a flow of air, which enters the first case portion 20, in the longitudinal direction E, a movement path of air passing through the first case portion 20 is shortened, and a larger amount of air may be purified within a short time. Thus, air cleaning efficiency may be improved.

Also, since the inlet portion 32 extends in the vertical direction at one side surface of the first case portion 20, the one side surface of the first case portion 20 is utilized as a space for suctioning air. Also, since the inlet portion 32 is formed along the one side surface of the first case portion 20 that has a curved shape, the number of inlet holes 34 is increased as compared to when the inlet portion 32 is installed along a straight line so as to be parallel to the filter portion 70. Since a suction flow rate of air increases according to the shape of the inlet portion 32, air cleaning efficiency may be improved.

The outlet portion 36 includes outlet grilles 37 that extend in the vertical direction, and outlet holes 38 are provided between the plurality of outlet grilles 37. The plurality of outlet grilles 37 may be installed at the other side surface of the housing portion 10 to form the outlet holes 38 for discharging air. Since a transverse cross-section of the outlet grilles 37 extends in the longitudinal direction E, the outlet holes 38 disposed between the outlet grilles 37 are also formed in the longitudinal direction E. Therefore, movement of air being discharged through the outlet portion 36 is guided in the longitudinal direction E along the outlet grilles 37.

Since the outlet holes 38 guide a flow of air, which is discharged to the outside of the first case portion 20, in the longitudinal direction E, the outlet holes 38 induce the movement path of air, which passes through the first case portion 20 as well as the inlet portion 32, to be shortened.

Also, since the outlet portion 36 extends in the vertical direction at the other side surface of the first case portion 20, most of the space of the other side surface of the first case portion 20 may be utilized as a space for discharging air. Also, since the outlet portion 36 is formed along the other side surface of the first case portion 20 that has a curved shape, the number of outlet holes 38 is increased as compared to when the outlet portion 36 is installed along a straight line so as to be parallel to the fan module portion 140. Since a discharge flow rate of air increases according to the shape of the outlet portion 36, air cleaning efficiency may be improved.

Also, the inlet holes 34 and the outlet holes 38 may be installed to face each other in the horizontal direction. Since the inlet holes 34 and the outlet holes 38 are installed at positions facing each other in the longitudinal direction E, air that enters through the inlet holes 34 is moved in the horizontal direction and then discharged to the outside of the first case portion 20 through the outlet holes 38. Therefore, the movement path of air passing through the first case portion 20 is induced to have the shortest possible length so that frictional resistance is minimized during movement of air, and an air blowing ability is improved to allow a larger amount of air to be purified within a short time. Thus, air cleaning efficiency may be improved. Also, power consumption for the operation of the fan module portion 140 may be reduced to reduce an electric bill.

Meanwhile, the length of the inlet portion 32 in the width direction W is shorter than the length of the filter portion 70 in the width direction W. Therefore, since air that enters toward the inlet portion 32 is purified while passing through the filter portion 70, air cleaning performance may be improved. In a case in which the length of the inlet portion 32 in the width direction W is less than or equal to the length of the filter portion 70 in the width direction W, air that enters through the inlet portion 32 may immediately move to the fan module portion 140 without passing through the filter portion 70, and thus an air caring ability may be degraded.

The first boss 42 and the second boss 44 which are formed as protrusions protruding to the inside of the first case body 30 are coupled to the filter case 80 to restrict movement of the filter case 80. The first boss 42 and the second boss 44 are installed inside the first case body 30 that faces the service door 50 and fix a first fixing protrusion 130 and a second fixing protrusion 132 of the filter case 80 which will be described below. Since the first boss 42 and the second boss 44 are installed at positions facing the service door 50, the first boss 42 and the second boss 44 are visible to a worker in a state in which the service door 50 is separated. Therefore, since the filter case 80, to which the first boss 42 and the second boss 44 are fastened, may be easily separated, it is possible to reduce the time and cost required for maintenance and repair work, including replacing the filter portion 70.

One side and the other side of the fan module portion 140 in the width direction W may be inserted into a first mounting groove portion 56 and the second mounting groove portion 40 such that movement thereof is restricted. The first mounting groove portion 56 is formed in the shape of a groove that extends in the vertical direction inside the service door 50, and the second mounting groove portion 40 is formed in the shape of a groove that extends in the vertical direction inside the first case body 30.

Since the first mounting groove portion 56 and the second mounting groove portion 40 are disposed at both sides of the outlet portion 36 in the width direction W, and both side corners of the fan module portion 140 are inserted into the first mounting groove portion 56 and the second mounting groove portion 40, the fan module portion 140 may be stably fixed. Also, since a separate bracket for fixing the fan module portion 140 is not used, the portable air caring apparatus 1 may be designed to have a small size, and the production costs and maintenance and repair costs may be reduced due to a decrease in the number of components.

The service door 50 is detachably installed at the first case body 30 and may be modified in various ways in which the service door 50 opens and closes the service space provided at the side surface of the first case body 30. The service door 50 according to an embodiment of the present invention may include at least any one of a door body 52, a gap maintaining protrusion 54, and the first mounting groove portion 56.

The door body 52 may be modified in various ways in which the door body 52 opens and closes the service space that is open at the side surface of the first case body 30. The door body 52 according to an embodiment of the present invention has a transverse cross-section formed in a curved shape and extends in the vertical direction. The door body 52 is detachably installed at a side surface of the first case portion 20 which is disposed between the inlet portion 32 and the outlet portion 36.

The gap maintaining protrusion 54 protrudes to the inside of the door body 52 and may be modified in various ways in which the gap maintaining protrusion 54 extends toward at least any one of the filter portion 70 and the filter case 80. The gap maintaining protrusion 54 according to an embodiment of the present invention is formed in the shape of a plate and protrudes to the inside of the door body 52 to come in contact with side surfaces of the filter case 80 and the filter portion 70 or be spaced a predetermined distance apart therefrom.

The gap maintaining protrusion may be provided as a plurality of gap maintaining protrusions, and the plurality of gap maintaining protrusions may come in contact with the side surfaces of the filter case 80 and the filter portion 70 or be spaced a predetermined distance apart therefrom. Therefore, the filter case 80 and the filter portion 70 are prevented from tilting in a direction toward the service door 50 to guide the filter case 80 and the filter portion 70 to be installed at set positions.

The first mounting groove portion 56 forms a groove in the vertical direction inside the door body 52. The first mounting groove portion 56 is formed in the vertical direction inside the door body 52 that comes in contact with a corner of the fan module portion 140. Also, the second mounting groove portion 40 forming a groove in the vertical direction is installed inside the first case body 30 that faces the first mounting groove portion 56.

The first mounting groove portion 56 and the second mounting groove portion 40 are installed at both sides of the outlet portion 36 in the width direction W. Therefore, both side corners of the fan module portion 140 in the width direction W are inserted into the first mounting groove portion 56 and the second mounting groove portion 40, and thus the fan module portion 140 may be stably mounted.

The second case portion 60 is connected to a lower portion of the first case portion 20 and may be modified in various ways in which a space, in which electronic components including the battery 220 are installed, is formed inside the second case portion 60.

A transverse cross-section of at least any one of the first case portion 20 and the second case portion 60 may be formed in a circular shape or an elliptical shape. Both the first case portion 20 and the second case portion 60 may be formed in a cylindrical shape or an elliptical shape, or only the second case portion 60 may be formed in the cylindrical or elliptical shape. Alternatively, as necessary, only the first case portion 20 may be formed in the cylindrical or elliptical shape.

In a case in which the second case portion 60 is formed in the cylindrical or elliptical shape and extends in the vertical direction H, it is convenient for a user to hold the outer periphery of the second case portion 60 with his or her hand, and the second case portion 60 may also be easily mounted on a cup holder of a vehicle that includes a groove portion having a substantially circular cross-section.

Also, in a case in which the first case portion 20 is formed in the cylindrical or elliptical shape, friction, which is generated when air moving in the horizontal direction by passing through the inside of the first case portion 20 comes in contact with the inside of the first case portion 20 formed in a curved shape, may be reduced such that a flow of air is facilitated.

Meanwhile, since an air flow path is formed inside the first case portion 20 while an air flow path is not formed inside the second case portion 60, suction and discharge of air through the first case portion 20 may be smoothly performed even when the second case portion 60 is inserted into a cup holder or held by a user's hand. Thus, convenience in use may be improved.

Also, since the second case portion 60 is mounted on a structure such as a cup holder having the shape of a groove which is concave toward the lower side, and air may be suctioned through the inlet portion 32 provided in the first case portion 20 without interfering with an external structure, air caring performance may be improved.

Also, since the transverse cross-section of the housing portion 10 is formed in the circular or elliptical shape, the housing portion 10 may be stably seated on the groove portion of the cup holder that has the circular transverse cross-section, and a separation distance between the housing portion 10 and the cup holder may be reduced such that the possibility of an occurrence of vibration and noise is reduced.

The first case portion 20 and the second case portion 60 may be separately formed and then assembled together or may be integrally formed. In this way, various modifications are possible.

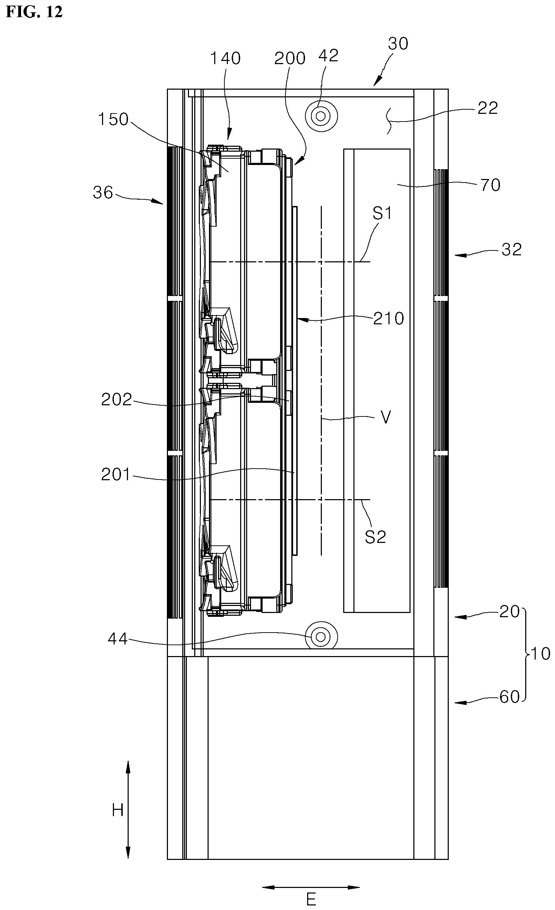

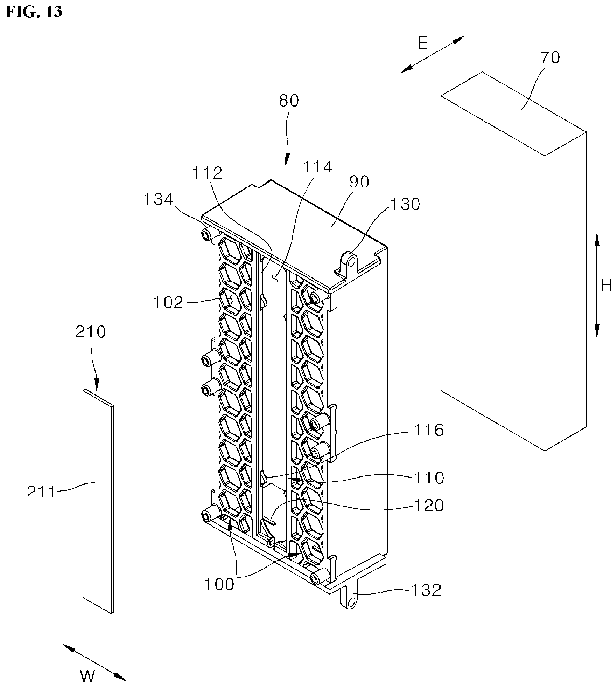

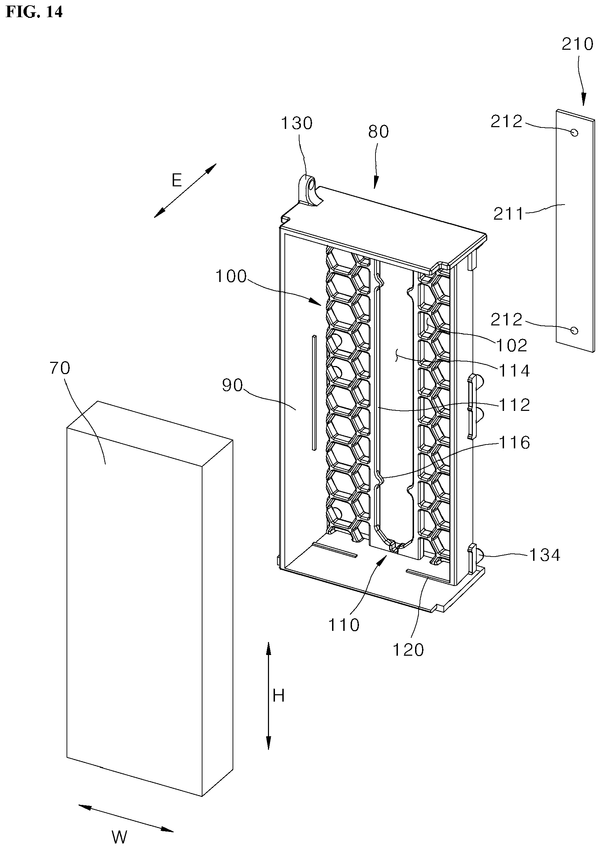

FIG. 12 is a front view illustrating a state in which the sanitizing portion 210 is installed between the fan module portion 140 and the filter portion 70 according to an embodiment of the present invention, and FIGS. 13 and 14 are perspective views illustrating a state in which the filter case 80 is disposed between the sanitizing portion 210 and the filter portion 70 according to an embodiment of the present invention.

As illustrated in FIGS. 12 to 14, the filter portion 70 is installed inside the first case portion 20 and may be modified in various ways in which the filter portion 70 purifies air that enters through the inlet portion 32. The filter portion 70 according to an embodiment of the present invention may be formed in a rectangular parallelepiped shape. Since the filter portion 70 is installed inside the filter case 80 and is easily separated from the filter case 80, the time taken to replace a filter may be reduced.

The filter portion 70 according to an embodiment of the present invention is installed inside the housing portion 10 facing the inlet portion 32. Since the filter portion 70 is disposed inside the housing portion 10 and installed in a state in which the filter portion 70 faces the inlet portion 32 and both sides of the filter portion 70 in the width direction W come in contact with the inside of the housing portion 10, air that enters through the inlet portion 32 may be guided to move toward the filter portion 70, and thus air caring performance may be improved.

Also, since the filter portion 70 is formed in a rectangular parallelepiped shape that extends in the vertical direction, an area of the filter portion 70 that comes in contact with air is increased, and thus air caring performance may be improved. Meanwhile, since the filter portion 70 is mounted inside the filter case 80 such that movement of the filter portion 70 is restricted, degradation of air caring performance due to movement of the filter portion 70 may be prevented.

According to the present embodiment, the inlet portion 32, the filter portion 70, the fan module portion 140, and the outlet portion 36 may be arranged in the first direction, and air flow may also occur in the same direction. That is, the air flow occurring due to the operation of the fan module portion 140 may occur in the same straight direction as the direction in which the inlet portion 32, the filter portion 70, the fan module portion 140, and the outlet portion 36 are arranged.

When the air flow occurs in the straight direction as described above, resistance to the air flow is lowered correspondingly, and thus the air flow may occur more smoothly. In this way, since suctioning a sufficient amount of air and discharging a sufficient amount of air, which corresponds to the amount of suctioned air, may be performed by the fan module portion 140, air caring performance of the portable air caring apparatus 1 may be improved correspondingly.

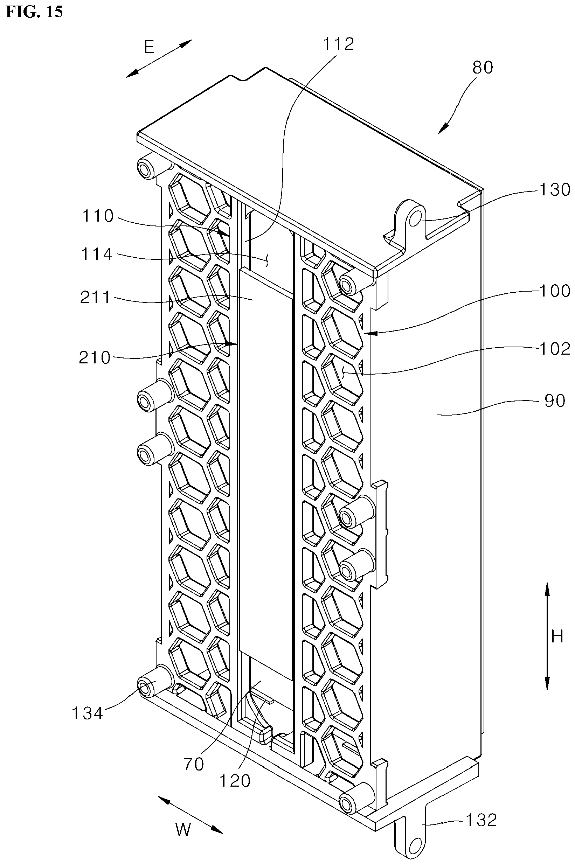

FIG. 15 is a perspective view illustrating the filter case 80 according to an embodiment of the present invention.

As illustrated in FIGS. 13 to 15, the filter case 80 and the filter portion 70 may be coupled and constitute a module, and in addition to such a configuration, the sanitizing portion 210 may be coupled to the filter case 80. That is, since the filter case 80, the filter portion 70, and the sanitizing portion 210 may constitute a single module, the time and cost for product assembly and maintenance and repair may be reduced.

Also, the filter portion 70 may be detachably installed in the filter case 80, and the filter case 80 may be disposed between the filter portion 70 and the fan module portion 140. Also, the filter case 80 may guide movement of air from the filter portion 70 to the fan module portion 140. Also, the filter case 80 may be modified in various ways in which the filter case 80 fixes the sanitizing portion 210 installed at a position spaced apart from the filter portion 70. The filter case 80 according to an embodiment of the present invention may include at least any one of a case main body portion 90, a flow path guiding portion 100, a fixing supporting portion 110, a catching step 120, the first fixing protrusion 130, the second fixing protrusion 132, and a first coupling protrusion 134.

The case main body portion 90 may be fixed to the inside of the housing portion 10 and may be modified in various ways in which the filter portion 70 is mounted inside the case main body portion 90. The case main body portion 90 forms a framework of the exterior of the filter case 80.

In the present embodiment, the case main body portion 90 is illustrated as being formed as a quadrilateral frame of which both sides in the longitudinal direction E are open. An insertion space for accommodating the filter portion 70 is formed inside the case main body portion 90. Also, the rear of the case main body portion 90 is open, and in this way, a path for inserting the filter portion 70 into the insertion space inside the case main body portion 90 is formed.

The filter portion 70 is mounted in the insertion space inside the case main body portion 90. A seating groove or the catching step 120 that allows the filter portion 70 to be firmly mounted inside the case main body portion 90 may be provided at an inner side surface of the case main body portion 90.

Both sides of the case main body portion 90 in the longitudinal direction E are open, and a length in the longitudinal direction E of a side surface disposed at one side of the case main body portion 90 in the width direction W that faces the service door 50 is shorter than a length in the longitudinal direction E of a side surface disposed at the other side of the case main body portion 90 in the width direction W. Therefore, in a case in which the service door 50 is separated from the first case body 30 and then the filter portion 70 is replaced, since the filter portion 70 may be easily withdrawn through one side surface of the case main body portion 90, the time taken to replace the filter portion 70 may be reduced.

The flow path guiding portion 100 is installed at one side of the case main body portion 90 and may be modified in various ways in which the flow path guiding portion 100 forms a path along which air moves from the filter portion 70 toward the fan module portion 140. The flow path guiding portion 100 according to an embodiment of the present invention is disposed between the filter portion 70 and the fan module portion 140 and forms a plurality of through-holes 102.

In a case in which a direction in which the filter portion 70 is disposed with respect to the filter case 80 is referred to as "rear" and a direction in which the outlet portion 36 is disposed with respect to the filter case 80 is referred to as "front," the flow path guiding portion 100 is connected to the case main body portion 90 which is disposed at the front of the filter portion 70. The through-holes 102 are formed to pass through in a front-rear direction. The through-holes 102 form a path for allowing air that has passed through the filter portion 70 to move in a direction toward the fan module portion 140. That is, the plurality of through-holes 102 are provided in the front surface of the filter case 80, and each through-hole 102 is formed to pass through in the front-rear direction at the front surface of the filter case 80.

The through-holes 102 may be formed in a hexagonal shape. Also, the plurality of through-holes 102 may be arranged in the shape of a honeycomb, and accordingly, a honeycomb structure may be formed at the front surface of the filter case 80.

Since the honeycomb structure is formed at the front surface of the filter case 80 as described above, in addition to securing a path for movement of air, rigidity of the filter case 80 may be secured, and weight reduction may be achieved.

The fixing supporting portion 110 may be disposed at the center of the flow path guiding portion 100 in the width direction W. Also, the fixing supporting portion 110 extends in the vertical direction and may be modified in various ways in which the fixing supporting portion 110 includes a protrusion that supports the sanitizing portion 210. The fixing supporting portion 110 according to an embodiment of the present invention may include a filter frame 112 and a fixing protrusion 116.

The filter frame 112 forms an inner hole 114 extending in the vertical direction, and both sides of the filter frame 112 in the width direction W are connected to the flow path guiding portion 100. Both sides of the filter frame 112 in the vertical direction H are connected to the case main body portion 90 and constitute a frame having the shape of a quadrilateral frame extending in the vertical direction. The shape of the inner hole 114 corresponds to the shape of the sanitizing portion 210. Therefore, the sanitizing portion 210 may be easily installed in a state of being inserted into the inner hole 114.

The fixing protrusion 116 is provided as a plurality of protrusions that protrude from the filter frame 112 toward the inner hole 114. The fixing protrusion 116 may be modified in various ways in which the fixing protrusion 116 restricts movement of the sanitizing portion 210 seated in the inner hole 114. The fixing protrusion 116 according to an embodiment of the present invention is disposed between the sanitizing portion 210, which is seated in the inner hole 114, and the filter portion 70 and prevents the sanitizing portion 210 from tilting in a direction toward the filter portion 70.

Since the flow path guiding portion 100 is installed at both sides of the fixing supporting portion 110, extends in the vertical direction, and includes the plurality of through-holes 102, air that has passed through the filter case 80 may move to the fan module portion 140.

The catching step 120 may be modified in various ways in which the catching step 120 protrudes to the inside of the case main body portion 90 and restricts movement of the filter portion 70. The catching step 120 according to an embodiment of the present invention is a band-shaped protrusion that protrudes to the inside of the case main body portion 90, and an installation position of the catching step 120 is set in consideration of an irradiation angle of the sanitizing portion 210. Since the catching step 120 is installed in the shape of a band along an inner periphery of the case main body portion 90, the filter portion 70 caught on the catching step 120 may be installed to be spaced a predetermined distance apart from the sanitizing portion 210. Therefore, the entire side surface of the filter portion 70, which faces the sanitizing portion 210, may be irradiated with the sanitizing light of the sanitizing portion 210 to improve the effect of sanitizing the filter portion 70.

The first fixing protrusion 130 may protrude to an upper side of the case main body portion 90 and be fixed to the first boss 42 provided inside the housing portion 10. Also, the second fixing protrusion 132 may protrude to a lower side of the case main body portion 90 and be fixed to the second boss 44 provided inside the housing portion 10. In a state in which the first fixing protrusion 130 faces the first boss 42, movement of the first fixing protrusion 130 may be restricted by fastening the first fixing protrusion 130 with a bolt. Also, in a state in which the second fixing protrusion 132 faces the second boss 44, movement of the second fixing protrusion 132 may be restricted by fastening the second fixing protrusion 132 with a bolt.

Meanwhile, since the first coupling protrusion 134, which protrudes in a direction from the case main body portion 90 toward the fan module portion 140, is inserted into the fan module portion 140, coupling between the filter case 80 and the fan module portion 140 may be easily performed, and as necessary, the filter case 80 and the fan module portion 140 may constitute a single module. The first coupling protrusion 134 is formed in the shape of a circular pipe and extends in the longitudinal direction E from the front of the case main body portion 90.

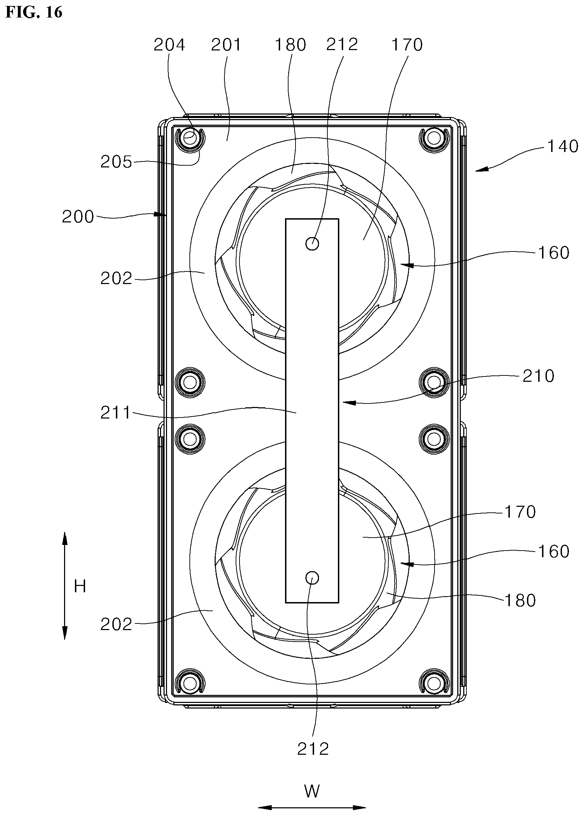

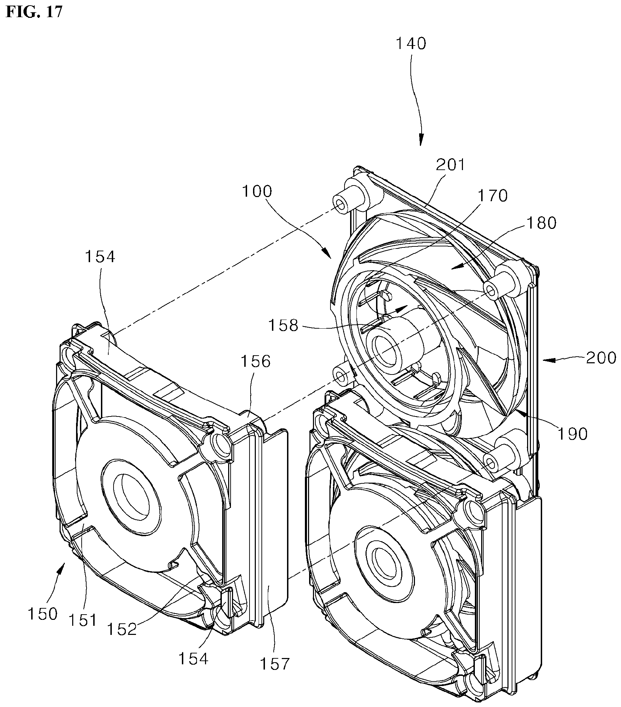

FIG. 16 is a view illustrating a state in which the sanitizing portion 210 is installed at the center of the fan module portion 140 according to an embodiment of the present invention, and FIG. 17 is a perspective view illustrating a state in which a fan housing 150 is separated from the fan module portion 140 according to an embodiment of the present invention.

As illustrated in FIGS. 12, 16, and 17, the fan module portion 140 is installed inside the housing portion 10 facing the outlet portion 36 and may be modified in various ways in which the fan module portion 140 suctions air from the rear of the portable air caring apparatus 1 and discharges air to the front.

The fan module portion 140 is accommodated in the accommodation space 22 of the housing portion 10 and may be disposed in the first area A. **In** the fan module portion 140, two fan housings 150 and two fan members 160 may constitute modules each including a single fan housing 150 and a single fan member 160, and the modules may be arranged in the vertical direction.

The fan module portion 140 may be disposed between the outlet portion 36 and the filter case 80, and more specifically, between the outlet portion 36 and the sanitizing portion 210. Therefore, the fan module portion 140 serves to blow air and restrict movement of the sanitizing portion 210 together with the filter case 80.

The length of the fan module portion 140 in the width direction W is formed to be longer than a length of a discharge portion in the width direction W. Therefore, the fan module portion 140 is fixed inside the first case portion 20 disposed at both sides of the discharge portion in the width direction W, and air discharged from the fan module portion toward the front is moved along an inner curved surface of the first case portion 20, which is disposed between the fan module portion 140 and the discharge portion, and then is discharged to the front of the portable air caring apparatus 1 through the discharge portion. That is, since the inner curved surface of the first case portion 20 disposed between the fan module portion 140 and the discharge portion serves as an air guide that guides movement of air toward the outlet portion 36, the ability of the fan module portion 140 to blow air in a straight line is improved.

Since both front corners of the fan module portion 140 are inserted into the first mounting groove portion 56 and the second mounting groove portion 40 that are provided inside the housing portion 10 and thus movement of the fan module portion 140 is restricted, the size of the first case portion 20 is not necessarily enlarged due to fixing or fastening the fan module portion 140. In this way, reduction of product size is possible. Also, for use in a vehicle, the portable air caring apparatus 1 according to the present invention may be implemented to have a size that allows the portable air caring apparatus 1 to be inserted into a cup holder.

The fan member 160 according to the present invention rotates due to operating a motor. Only a rotating shaft of the motor that rotates the fan member 160 may be connected to the fan member 160, a rotor may be installed at the fan member 160, and a stator may be installed in the fan housing 150 whose rotation is restricted. Since a magnetic field of the stator changes, the shaft that rotates along with the rotor may be connected to the fan member 160, and the rotor and the fan member 160 may rotate about the stator. Since the configuration of the motor rotating the fan member 160 is a known configuration, detailed description thereof will be omitted.

The fan module portion 140 according to an embodiment of the present invention may include the fan housing 150, the fan member 160, and a fan base 200.

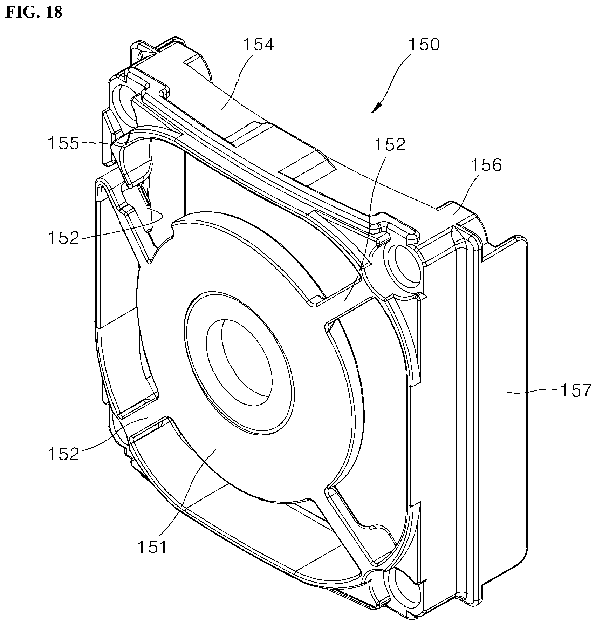

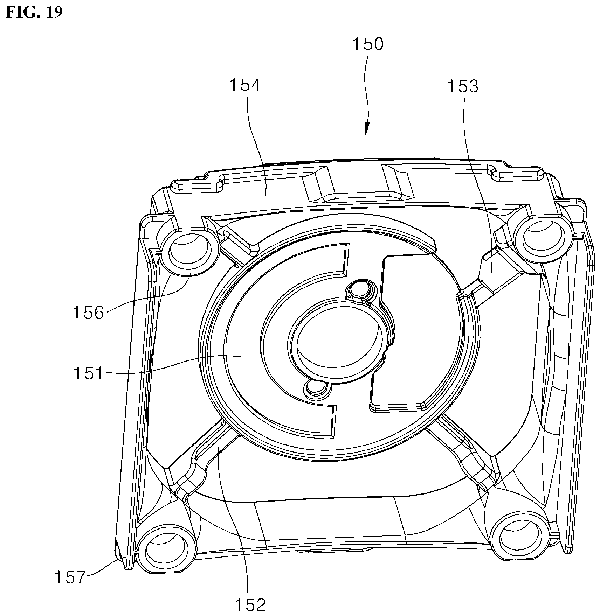

FIG. 18 is a perspective view illustrating the fan housing 150 according to an embodiment of the present invention, and FIG. 19 is a perspective view illustrating the inside of the fan housing 150 according to an embodiment of the present invention.

As illustrated in FIGS. 17 to 19, the fan housing 150 is fixed to the inside of the housing portion 10 and may be modified in various ways in which the fan housing 150 has a space formed therein to allow rotation of the fan member 160.

Also, the fan housing 150 may surround the fan member 160 and be coupled to the fan base 200. Also, since the fan housing 150, the fan member 160, and the fan base 200 may constitute a module, product assembly and maintenance and repair may be performed conveniently.

The fan housing 150 according to an embodiment of the present invention may include at least any one of a support plate 151, a connection support 152, a wire guide 153, a side support portion 154, a wire fixing protrusion 155, a protruding boss 156, a side blade 157, and a central guide 158.

The support plate 151 is installed in the shape of a plate at a position facing the fan member 160, and a hole is formed at the center of the support plate 151. A motor may be installed at the center of the support plate 151, or a shaft connected to the motor may be installed in the first direction. The support plate 151 is disposed at the rear of the outlet portion 36.

The connection support 152 extends to the outside of the support plate 151 and is connected to the side support portion 154. The connection support 152 according to an embodiment of the present invention may be provided as a plurality of connection supports 152 and may be installed in the shape of a rod. The connection supports 152 extending radially outward from the support plate 151 are connected to the side support portion 154 having the shape of a frame.

The wire guide 153 is continuously installed on the connection support 152 and supports a wire installed along the side surface of the connection support 152. The wire guide 153 is formed in the shape of a protrusion, which is disposed at the rear of the side surface of the connection support 152, and guides a wire of the motor installed on the support plate 151 to be installed to extend to the outside of the fan housing 150. The wire guide 153 may be installed at the side surface of the connection support 152 and form a concave groove to allow the wire to be disposed therein. Therefore, since the wire installed in the wire guide 153 is disposed in the concave groove disposed at the side surface of the connection support 152, and the rear of the wire is supported by the wire guide 153, damage to the wire may be prevented.

The side support portion 154 may be modified in various ways in which the side support portion 154 forms a circular or quadrilateral frame along the periphery of the support plate 151. The side support portion 154 according to an embodiment of the present invention is formed in the shape of a quadrilateral frame in which both sides in the first direction are open. The outer side of the side support portion 154 comes in contact with the inside of the first case portion 20, and the inner side of the side support portion 154 is connected to the connection support 152.

The protruding boss 156 extends to the rear of the side support portion 154 toward the fan base 200 and may be modified in various ways in which the protruding boss 156 includes a groove portion for allowing a coupling protrusion 204 of the fan base 200, which will be described below, to be inserted thereinto. The protruding boss 156 according to an embodiment of the present invention is provided as a plurality of protruding bosses 156 installed along the periphery of the side support portion 154.

The wire fixing protrusion 155 protrudes from the side support portion 154 and may be modified in various ways in which the wire fixing protrusion 155 is disposed in a lateral direction of a wire, which is disposed outside the connection support 152, to restrict detachment of the wire. The wire fixing protrusion 155 according to an embodiment of the present invention may be disposed at the front of a wire, which is guided to a corner of the fan housing 150 along the connection support 152, and may restrict the wire from protruding to the front of the fan housing 150 to prevent damage to the wire.

The side blade 157 is connected to both sides of the side support portion 154 in the width direction W and is formed in the shape of a plate extending in the first direction. The side blade 157 extends in the vertical direction H and extends to the rear of the side support portion 154. Also, the side blade 157 serves as an air guide that allows air moved by the fan member 160 to move in a direction toward the outlet portion 36 and serves to prevent the fan member 160 rotating at a high speed from colliding with another object.

The central guide 158 is formed in the shape of a circular pipe and is fixed to the support plate 151. A hollow formed inside the central guide 158 extends in the first direction. Since the shaft connected to the fan member 160 is disposed at an inner side of the central guide 158, the central guide 158 serves to guide stable rotation of the shaft.

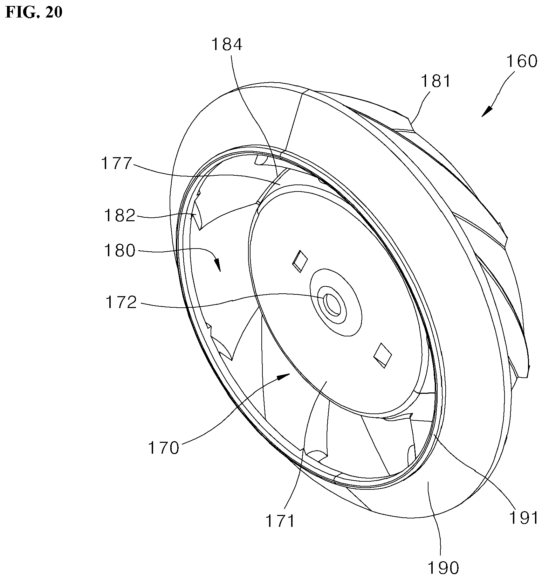

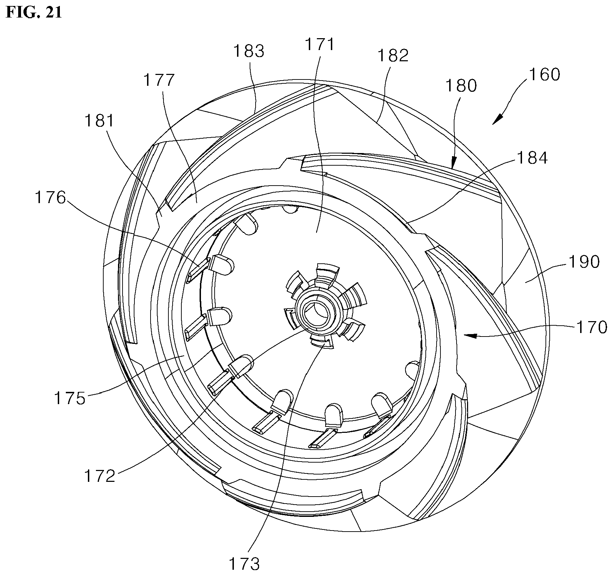

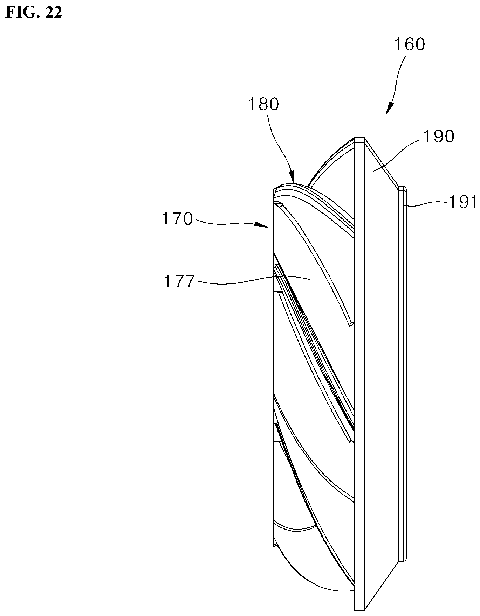

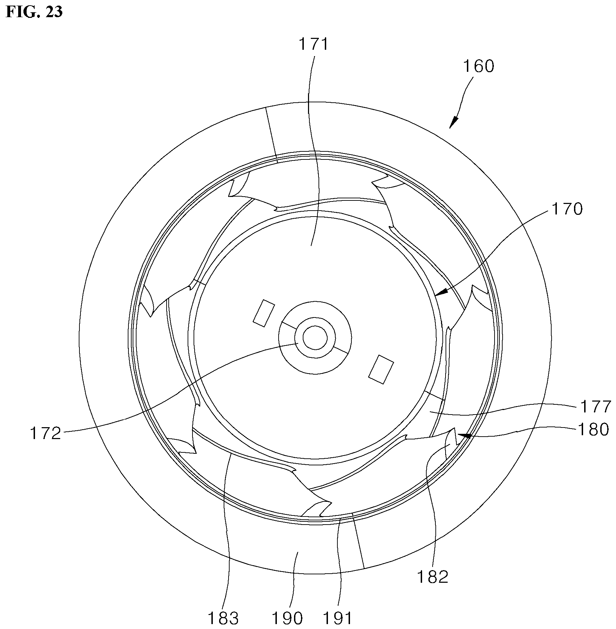

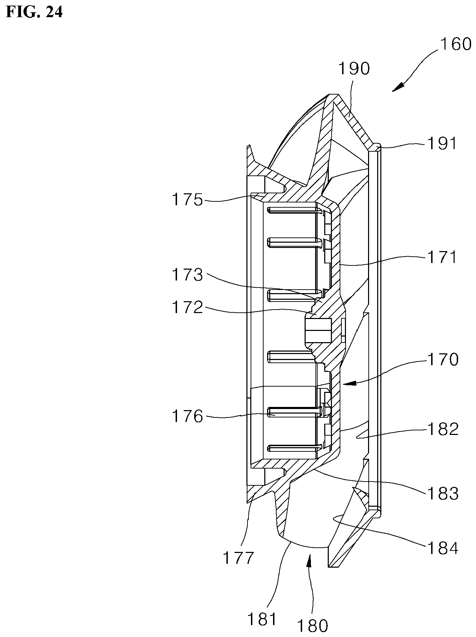

FIG. 20 is a perspective view illustrating a shroud 190 of the fan member 160 according to an embodiment of the present invention, FIG. 21 is a perspective view illustrating an axial coupling portion 172 of the fan member 160 according to an embodiment of the present invention, FIG. 22 is a front view of the fan member 160 according to an embodiment of the present invention, FIG. 23 is a view illustrating a hub 170 of the fan member 160 according to an embodiment of the present invention, and FIG. 24 is a cross-sectional view of the fan member 160 according to an embodiment of the present invention.

As illustrated in FIGS. 20 to 24, the fan member 160 is rotatably installed inside the fan housing 150 and may be modified in various ways in which the fan member 160 is able to move air in the direction toward the outlet portion 36.

A diagonal flow fan may be used as the fan member 160, but this is only an embodiment of the present invention, and other types of fans may also be used as the fan member 160 according to the present invention. The fan member 160 according to an embodiment of the present invention may include at least any one of the hub 170, a fan blade 180, and the shroud 190.

The hub 170 is disposed at the center of the fan housing 150 and may be modified in various ways in which the hub 170 receives external power and rotates.

The hub 170 is disposed at the center of the fan member 160 in the radial direction and may rotate along with the rotor and the shaft, which is an output shaft of the motor, that constitute the motor. The hub 170 according to an embodiment of the present invention may include at least any one of a hub plate portion 171, the axial coupling portion 172, an inner side protruding portion 175, and a skirt portion 177.

The hub plate portion 171 may be formed in the shape of a disk that is parallel to the support plate 151. The axial coupling portion 172 may be provided on the hub plate portion 171. The axial coupling portion 172 may be disposed at the center of the hub plate portion 171 in the radial direction. The axial coupling portion 172 may be formed to protrude from the hub plate portion 171 in the first direction.

The axial coupling portion 172 may be coupled to an end portion of a shaft that transmits rotary power. For example, coupling between the axial coupling portion 172 and the shaft may be performed in a form in which the shaft is fitted and fixed to the axial coupling portion 172.

First reinforcing protrusions 173 are installed at predetermined intervals along the outer periphery of the axial coupling portion 172. The first reinforcing protrusions 173 are radially installed about the center of the axial coupling portion 172 and are formed as band-shaped protrusions at an outer side of the axial coupling portion 172. Therefore, since stress concentrated on the axial coupling portion 172 is distributed through the first reinforcing protrusions 173, structural rigidity of the axial coupling portion 172 may be improved.

The inner side protruding portion 175 may protrude in a direction from the hub plate portion 171 toward where the support plate 151 is installed. The inner side protruding portion 175 according to an embodiment of the present invention is installed in an arc shape along the outer side edge of the hub plate portion 171. The inner side protruding portion 175 is formed in the shape of a pipe that extends in the first direction.

Also, second reinforcing protrusions 176 are installed at predetermined intervals along the inner periphery of the inner side protruding portion 175. The second reinforcing protrusions 176 are installed in the first direction along the inner side surface of the inner side protruding portion 175, and the second reinforcing protrusions 176 are formed as band-shaped protrusions that extend toward the axial coupling portion 172. Therefore, since stress concentrated on the inner side protruding portion 175 is distributed through the second reinforcing protrusions 176, structural rigidity of the inner side protruding portion 175 may be improved. As necessary, the rotor of the motor may be fixed to the inner side of the inner side protruding portion 175.

The skirt portion 177 may protrude in a direction from an edge of the hub plate portion 171 toward the support plate 151. The skirt portion 177 may form an inclined surface that is further inclined radially outward away from the hub plate portion 171 in the first direction. The skirt portion 177 is disposed at an outer side of the inner side protruding portion 175, and an inner diameter of the skirt portion 177 gradually increases in a direction toward the front of the hub plate portion 171.

For example, the hub plate portion 171 and the skirt portion 177 may be connected in the shape of a truncated cone in which a hollow is formed and whose one side is open. The skirt portion 177 may be formed in the shape of a funnel in which the front is open and the rear is blocked by the hub plate portion 171.

The shroud 190 is connected to an end portion of the fan blade 180 and is installed in an annular shape and may be modified in various ways in which the shroud 190 may be spaced apart from the fan base 200.

The shroud 190 is installed along the outer periphery of the skirt portion 177, and the shroud 190 and the skirt portion 177 are connected by the fan blade 180. Also, an outer diameter of the hub 170 and an inner diameter of the shroud 190 may gradually decrease in a direction from the outlet portion 36 toward the inlet portion 32.

The shroud 190 may be spaced a predetermined distance apart from the hub 170 in the radial direction of the fan module portion 140 and may be disposed at an outer side of the hub 170 in the radial direction. Also, the shroud 190 may be spaced apart from the hub 170 as much as a distance that corresponds to a length of the fan blade 180 in the radial direction. Also, each fan blade 180 may connect the skirt portion 177, which is disposed at the hub 170, and the shroud 190.

The shroud 190 may form an inclined surface that is substantially parallel to the skirt portion 177. **In** the present embodiment, the skirt portion 177 and the shroud 190 are arranged in a form in which a distance between the skirt portion 177 and the shroud 190 gradually increases in a direction toward the front of the shroud 190. **In** the present invention, the front refers to a direction from the center of the housing portion 10 toward the outlet portion 36, and the rear refers to a direction from the center of the housing portion 10 toward the inlet portion 32.

An inlet protrusion 191 provided at the rear of the shroud 190 is a ring-shaped protrusion and extends in the first direction from the funnel-shaped shroud 190. Since the inlet protrusion 191 is disposed at an inner side of a bell mouth 202 which will be described below, the inlet protrusion 191 may prevent the returning flow of air that enters through an inlet provided in the shroud 190 along an outer side of the shroud 190.

The fan blade 180 may be provided as a plurality of fan blades 180, and the plurality of fan blades 180 may be disposed to be spaced apart from each other at equal intervals along an outer peripheral surface of the hub 170. The fan blades 180 protrude to the outside of the hub 170 with respect to the center of the hub 170 and extend in a spiral shape. Also, the plurality of fan blades 180 may be disposed to be spaced apart from each other at predetermined intervals in a peripheral direction of the hub 170.

The fan blades 180 according to an embodiment of the present invention may protrude to the outside of the skirt portion 177 in a centrifugal direction extending in a spiral shape from the center of the axial coupling portion 172. Also, when a direction from the outside of the axial coupling portion 172 toward the axial coupling portion 172 is the radial direction, the inner side of the fan blades 180 in the radial direction may be connected to the skirt portion 177, and the outer side of the fan blades 180 in the radial direction may be connected to the shroud 190 which will be described below.

The skirt portion 177 is a portion of the hub 170 that is directly connected to the fan blades 180 and is a portion that also comes in direct contact with air passing through the fan blades 180. The skirt portion 177 is also closely related to a flow path of air passing through the fan module portion 140.

Each fan blade 180 that connects between the shroud 190 and the skirt portion 177 may include a first end portion 181, a second end portion 182, a first edge 183, and a second edge 184.

The first end portion 181 is disposed at a front end of the fan blade 180 in a rotational direction thereof and may be formed in a linear shape that extends in the radial direction. The rotational direction is defined as a direction in which rotation of the fan member 160 occurs.

The second end portion 182 is disposed at a rear end of the fan blade 180 in the rotational direction thereof and may be radially formed about the axial coupling portion 172.

The first edge 183 may connect one end of the first end portion 181 and one end of the second end portion 182. The first edge 183 may be connected to an inner peripheral surface of the shroud 190.

The second edge 184 may connect the other end of the first end portion 181 and the other end of the second end portion 182. The second edge 184 may be connected to the outer peripheral surface of the hub 170.

That is, the one end of the first end portion 181 and the one end of the second end portion 182 may be connected to the inner peripheral surface of the shroud 190. Also, the other end of the first end portion 181 and the other end of the second end portion 182 may be connected to an outer peripheral surface of the skirt portion 177.

The one end of the first end portion 181 may be disposed closer to the center of the hub plate portion 171 in the radial direction than the one end of the second end portion 182. Also, the other end of the second end portion 182 may be disposed closer to the center of the hub plate portion 171 in the radial direction than the other end of the first end portion 181. This is because the one end and the other end of the first end portion 181 are disposed more toward the front in the rotational direction than the one end and the other end of the second end portion 182, and the skirt portion 177 is formed such that a radius thereof gradually decreases toward the front in the rotational direction.

According to the present embodiment, the fan blade 180 is connected to the skirt portion 177 of the hub 170. In order to guide a flow of air entering the fan module portion 140 in a direction that is inclined upward, the skirt portion 177 forms an inclined surface that is inclined upward.

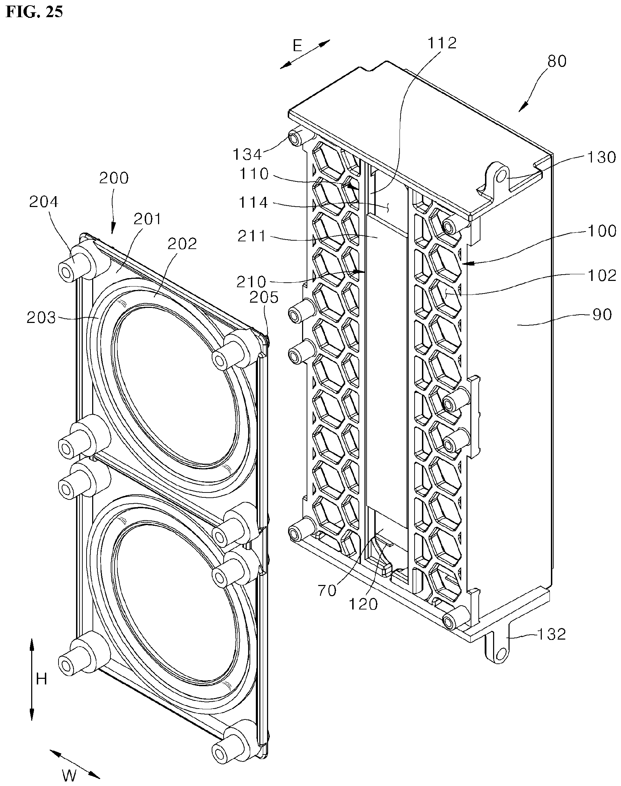

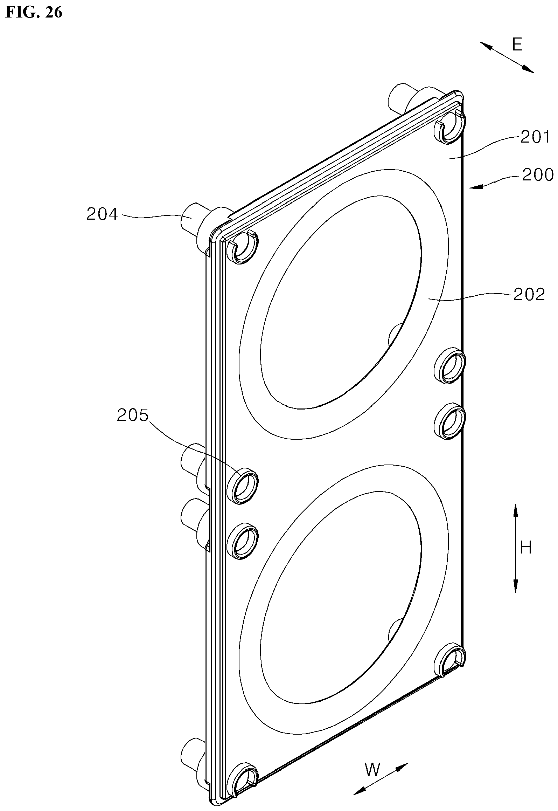

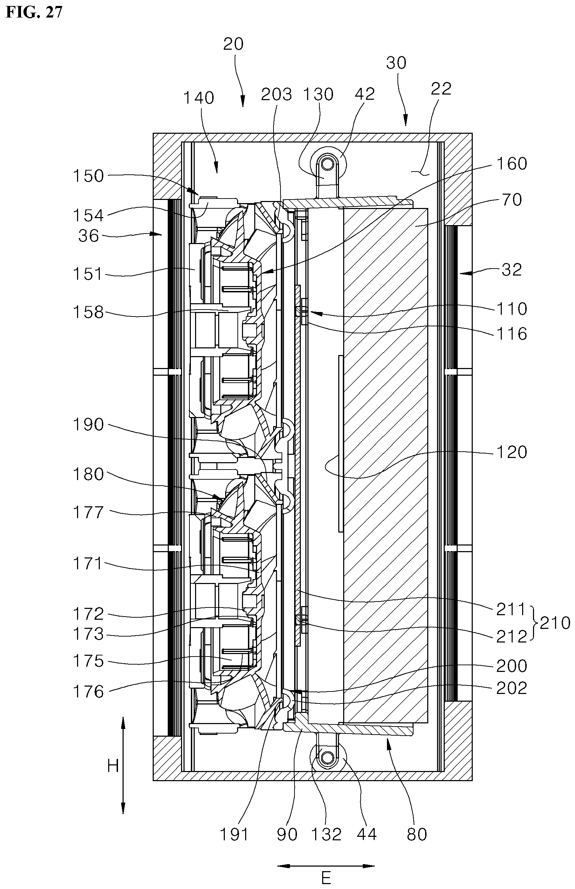

FIG. 25 is a perspective view illustrating a state in which the fan base 200 is separated from the filter case 80 according to an embodiment of the present invention, FIG. 26 is a perspective view illustrating the fan base 200 according to an embodiment of the present invention, and FIG. 27 is a view illustrating a state in which the sanitizing portion 210 is installed in a state of being spaced apart from the filter portion 70 according to an embodiment of the present invention.

As illustrated in FIGS. 25 to 27, the fan base 200 is coupled to the rear of the fan housing 150 and may be modified in various ways in which the fan base 200 guides air, which has passed through the filter portion 70, to enter the fan member 160.

The fan base 200 may be disposed between the filter portion 70 and the fan member 160. The front of the fan base 200 is coupled to the fan housing 150, and the rear of the fan base 200 is coupled to the filter case 80. Also, the fan base 200 may support the front of the sanitizing portion 210, and the filter case 80 may support the rear of the sanitizing portion 210. Also, a movement path of air being suctioned into the fan member 160 may be formed in the fan base 200, and as many air movement paths as the number of fan members 160 may be formed.

The fan base 200 is formed in a substantially plate-like shape, and an outer shape of the fan base 200 may be formed to correspond to the shape of the fan housing 150. For example, in a case in which the fan housing 150 has a quadrilateral shape and is continuously installed in the vertical direction, the fan base 200 coupled to the fan housing 150 may also be formed in the shape of a rectangular plate that extends in the vertical direction. The fan base 200 according to an embodiment of the present invention may include at least any one of a base plate 201, the bell mouth 202, the coupling protrusion 204, and a protruding rib 203.

The base plate 201 may be disposed between the filter case 80 and the fan member 160. The base plate 201 is formed in the shape of a rectangular plate that extends in the vertical direction H and has a hollow formed at the center to allow movement of air. The hollow is provided as a plurality of hollows, and the plurality of hollows are arranged in the vertical direction H.

The bell mouth 202 is installed in an annular shape at an inner side of the base plate 201 that faces the hollows. The bell mouth 202 extends in the circumferential direction and has a longitudinal end surface formed in a concave shape that surrounds an end portion of the inlet protrusion 191 of the shroud 190.

The bell mouth 202 may be formed in a shape that surrounds an outer peripheral surface of the hollows formed at the center of the base plate 201. The bell mouth 202 is formed to be convex toward the rear and may form a groove portion that is concave toward the front.

At least one portion of the bell mouth 202 may be inserted into the shroud 190 in the radial direction. The bell mouth 202 may guide a suctioning flow at the inlet of the fan module portion 140 to contribute to an improvement in suctioning and discharging performance of the fan module portion 140. Also, since the rear of the bell mouth 202 is formed as a convex curved surface, an increase in frictional resistance of air moving forward through the bell mouth 202 may be decreased, and thus air blowing may be facilitated.

Also, the coupling protrusion 204 protrudes to the front from the base plate 201 in the first direction and is coupled to the groove portion of the protruding boss 156, which is disposed in the fan housing 150, by being fitted thereto to fix the fan base 200 to the fan housing 150. The fan base 200 and the fan housing 150 may be coupled at a plurality of points due to coupling performed between the coupling protrusion 204 and the protruding boss 156. When coupling between the fan base 200 and the fan housing 150 is performed as described above, the fan member 160 may be rotatably installed between the fan base 200 and the fan housing 150.

The protruding rib 203 is installed in an annular shape that surrounds the outer periphery of the bell mouth 202 and may be installed to be parallel to the outer side of the fan member 160. Also, the protruding rib 203 protrudes from the base plate 201 and may be disposed at the outer side of the bell mouth 202 in the radial direction.

Also, the protruding rib 203 may be integrally formed with the base plate 201. More specifically, the base plate 201, the bell mouth 202, and the protruding rib 203 may be integrally formed.

Also, the protruding rib 203 may be installed to be inclined at the same angle as an outer side surface of the shroud 190, and a distance between the protruding rib 203 and the shroud 190 may be maintained to be constant. The protruding rib 203 may protrude in a shape forming an inclined surface. The inclined surface of the protruding rib 203 may be formed as an inclined surface that is spaced a predetermined distance apart from the shroud 190 and is parallel to the inclined surface of the shroud 190.

Also, the inclined surface of the protruding rib 203 may have the same angle of inclination as an inclined surface of an inner side guiding portion disposed in the fan housing 150. Therefore, it is possible to prevent a return air phenomenon in which a portion of air, which has moved to the front through a space between the shroud 190 and the skirt portion 177, moves to the inlet of the fan member 160 through a space between the shroud 190 and the protruding rib 203. Therefore, an occurrence of flow loss due to the return air phenomenon may be suppressed, and since the rigidity of the fan base 200 is increased, an effect of suppressing deformation of the fan base 200 due to an external force may be provided.

Meanwhile, a ring-shaped coupling guiding protrusion 205 may be installed at the rear of the base plate 201 that faces the protruding rib 203. The first coupling protrusion 134, which protrudes to the front of the filter case 80, is moved to an inner side of the coupling guiding protrusion 205 and then inserted to the rear of the coupling protrusion 204 and fixed by being fitted thereto.

In a case in which the first coupling protrusion 134 of the filter case 80 is moved to the inner side of the coupling protrusion 204 of the fan base 200 and coupled thereto without the coupling guiding protrusion 205, an area in which the first coupling protrusion 134 comes in contact with the fan base 200 may be reduced and a coupling force may be decreased, and thus the filter case 80 and the fan base 200 may be separated.

In order to allow compact design by suppressing an increase in the length of the fan base 200 in the longitudinal direction E while addressing the above problem, the coupling guiding protrusion 205 is additionally installed. When the first coupling protrusion 134 is coupled to the fan base 200, since the coupling guiding protrusion 205 and the inner side of the coupling protrusion 204 come in contact with the outer side surface of the first coupling protrusion 134, the area in which the first coupling protrusion 134 comes in contact with the fan base 200 is increased, and thus, the coupling force may be increased.

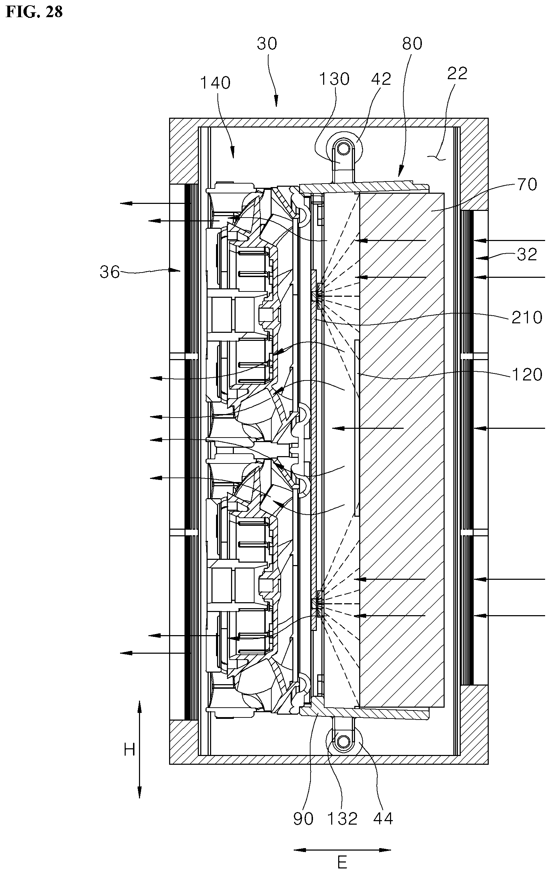

FIG. 28 is a view illustrating a state in which the sanitizing light is irradiated from the sanitizing portion 210 toward the filter portion 70 according to an embodiment of the present invention.

As illustrated in FIG. 28, the sanitizing portion 210 is mounted on the filter case 80 and may be modified in various ways in which the sanitizing portion 210 irradiates the sanitizing light toward the filter portion 70.

The sanitizing portion 210 according to an embodiment of the present invention is inserted into the filter frame 112, and movement of the sanitizing portion 210 may be restricted due to the sanitizing portion 210 being caught on the fixing protrusion 116. An installation position of the sanitizing portion 210 may be changed in various ways for sanitizing the filter portion 70 to prevent contamination of air while minimizing an increase in resistance of an air flow path.

As illustrated in FIG. 15, the sanitizing portion 210 is installed at the center of the filter case 80 in the width direction W and may extend in the vertical direction.

Also, as illustrated in FIG. 16, the sanitizing portion 210 may be disposed at the center of the fan module portion 140 in the width direction W. That is, since the front of the sanitizing portion 210 is disposed at the center of the fan module portion 140 in the width direction W and the rear of the sanitizing portion 210 is disposed at the center of the filter case 80 in the width direction W, the position of the sanitizing portion 210 is an optimal position that is set in consideration of the irradiation distance and irradiation angle of the sanitizing light while minimizing friction with air that moves from the filter case 80 to the fan module portion 140.

In an embodiment of the present invention, two fan members 160 may be installed in the vertical direction along the sanitizing portion 210. The upper end of the sanitizing portion 210 is installed at a lower position than the upper end of the hub 170 of the fan member 160 disposed at the upper side of the sanitizing portion 210. Therefore, since the sanitizing portion 210 does not interfere with an air suction flow path that is disposed at the upper side of the upper end of the hub 170 of the fan member 160 disposed at the upper side of the sanitizing portion 210, an increase in frictional resistance of the air flow path may be minimized.

Also, the lower end of the sanitizing portion 210 is installed at a higher position than the lower end of the hub 170 of the fan member 160 disposed at the lower side of the sanitizing portion 210. Therefore, since the sanitizing portion 210 does not interfere with an air suction flow path that is disposed at the lower side of the lower end of the hub 170 of the fan member 160 disposed at the lower side of the sanitizing portion 210, an increase in frictional resistance of the air flow path may be minimized.

In a state in which the plurality of fan members 160 are continuously installed in the vertical direction, since the upper end and lower end of the sanitizing portion 210 extending in the vertical direction do not protrude to the outside of the hub 170 of the fan member 160, interference of the sanitizing portion 210 with an air flow path along which air is suctioned into the fan member 160 may be minimized. Therefore, an increase in resistance of the air flow path along which air is suctioned into the fan member 160 may be minimized, and air caring performance may be improved.

The sanitizing portion 210 according to an embodiment of the present invention may include a printed circuit board (PCB) 211 and a sanitizing light source 212. The PCB 211 is a rectangular plate extending in the vertical direction, and the front of the PCB 211 is supported by the bell mouth 202 of the fan base 200 while the rear of the PCB 211 is supported by the fixing protrusion 116 of the filter case 80.

The sanitizing light source 212 may be an ultraviolet-C light emitting diode (UVC LED), or various other types of sanitizing apparatuses may be used as the sanitizing light source 212 in which the sanitizing light source 212 sterilizes the filter portion 70. The sanitizing light source 212 is installed at both sides of the PCB 211 in the vertical direction and irradiates the filter portion 70 with the sanitizing light.

The installation position and number of sanitizing light sources 212 may be changed in consideration of the length of the filter portion 70 in the vertical direction H according to the present invention. An irradiation angle of the sanitizing light irradiated by the sanitizing light source 212 is in a range of 140° to 160°. Therefore, since the rays of sanitizing light irradiated by the sanitizing light sources 212 installed at the upper side and lower side of the PCB 211 reach the filter portion 70 and partially overlap each other, the entire side surface located at the front of the filter portion 70 may be sanitized.

As illustrated in FIGS. 12 and 27, as the fan housing 150 and the fan member 160 according to an embodiment of the present invention, two fan housings 150 and two fan members 160 may be continuously installed in the vertical direction of the fan base 200, and a central axis of rotation of the fan member 160 may intersect the sanitizing portion 210 at a right angle.

A virtual line that passes through the center of rotation of the fan member 160 disposed at the upper side and extends in the first direction is set as a first horizontal center line P1, and a virtual line that passes through the center of rotation of the fan member 160 disposed at the lower side and extends in the first direction is set as a second horizontal center line P2.

When a virtual line that intersects the first horizontal center line P1 and the second horizontal center line P2 in the vertical direction is referred to as "vertical virtual line V," the sanitizing portion 210 is installed to be parallel to the vertical virtual line V. In a case in which the sanitizing portion 210 is installed to be inclined with respect to the vertical virtual line V, since a contact area with air passing through the filter portion 70 and moving to the fan module portion 140 increases and thus frictional resistance generated during movement of air is increased, there is a problem in that air cleaning performance is degraded.

However, in the case in which the sanitizing portion 210 is installed to be parallel to the vertical virtual line V, since the contact area with air passing through the filter portion 70 and moving to the fan module portion 140 may be minimized and thus an increase in the frictional resistance generated during movement of air may be minimized, degradation of air cleaning performance may be prevented.

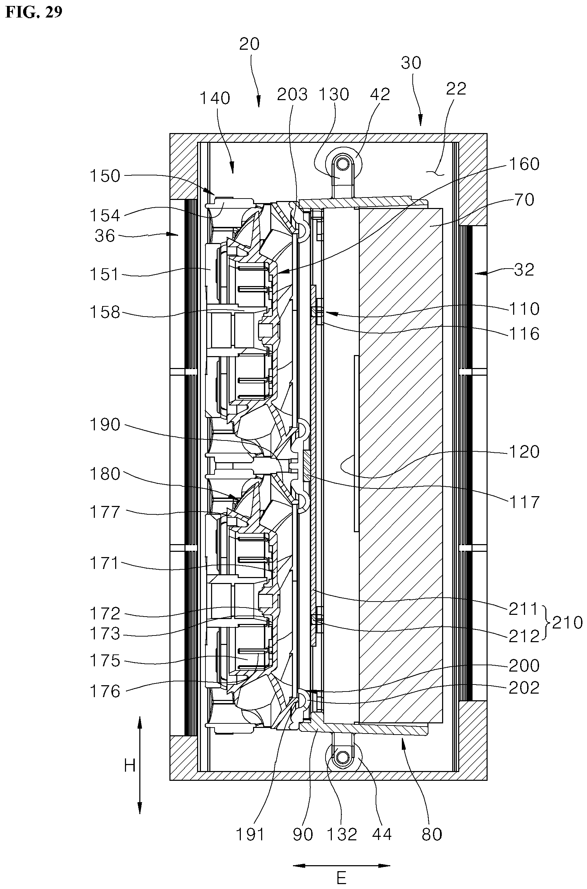

FIG. 29 is a view illustrating a state in which a locking protrusion 117 is installed on a rear surface of the sanitizing portion 210 according to an embodiment of the present invention. As illustrated in FIG. 29, a separate locking protrusion 117 may be additionally installed at the front of the sanitizing portion 210. In a state in which the sanitizing portion 210 is inserted into the filter frame 112 disposed in the filter case 80, the fixing protrusion 116 and the locking protrusion 117 protrude from the filter frame 112.

The fixing protrusion 116 is disposed at the rear of the sanitizing portion 210, and the locking protrusion 117 is disposed at the front of the sanitizing portion 210. Since the fixing protrusion 116 is disposed at the upper side and lower side of the sanitizing portion 210, and the locking protrusion 117 is disposed at the center of the sanitizing portion 210 and made of a plastic material such that its shape is deformable to some extent, the shapes of the fixing protrusion 116 and the locking protrusion 117 may be deformed when the sanitizing portion 210 is mounted. Also, after the mounting of the sanitizing portion 210 is completed, the shapes of the fixing protrusion 116 and the locking protrusion 117 may be restored to original shapes.

### [Aspects of air flow of portable air caring apparatus]

As illustrated in FIGS. 11 and 28, since air that enters through the inlet portion 32 is guided to move in the horizontal direction and is discharged to the outside of the housing portion 10 through the outlet portion 36 after sequentially passing through the filter portion 70, the filter case 80, and the fan module portion 140, a movement path of air is shortened, and thus air caring performance may be improved.

Also, since the sanitizing portion 210 is fixed in a shape of being inserted into the filter case 80, heat dissipation of the sanitizing portion 210 may be easily performed by air moving to the fan module portion 140 through the filter case 80, and thus the durability of the sanitizing portion 210 may be improved.

Also, since an area in which air enters the housing portion 10 through the inlet portion 32 is larger than an area of the filter portion 70 that faces the inlet portion 32, a large amount of air may move to the fan module portion 140 through the filter portion 70 even during a low-speed operation of the fan module portion 140. Thus, air cleaning efficiency may be improved.

Also, since the filter case 80 is installed to surround the outer side edge of the filter portion 70, air passing through the filter portion 70 does not move in the first direction toward the inlet portion 32 and is blocked from moving in the vertical direction H of the filter portion 70 through the edge of the filter portion 70. Thus, an ability of air to move in a straight line may be secured.

Also, since the sanitizing portion 210 is installed at the center of the filter case 80 and the fan module portion 140 in the width direction W, and the upper end and lower end of the sanitizing portion 210 do not protrude to the outside of the hub 170 which is installed at the upper side and lower side of the sanitizing portion 210, an increase in resistance of a flow path due to the sanitizing portion 210 may be minimized. Thus, suctioning, discharging, and filtering of air may be effectively performed while flow loss of air is minimized.

Meanwhile, physical particles such as dust, fine dust, and ultrafine dust, chemical substances such as odorous particles and harmful gases, and microorganisms such as bacteria and viruses that are contained in air may be filtered as the air passes through the filter portion 70.

Also, since the center of the inlet portion 32 in the width direction W, the center of the filter portion 70 in the width direction W, the center of the filter case 80 in the width direction W, the center of the sanitizing portion 210 in the width direction W, the center of the fan module portion 140 in the width direction W, and the center of the outlet portion 36 in the width direction W are arranged in a straight line along the second horizontal center line P2, suctioning and filtering of air may be effectively performed while flow loss of air is minimized.

Air that has passed through the filter portion 70, that is, purified air, may enter the fan module portion 140. A flow of air may be guided by the bell mouth 202, and in this way, air may be effectively guided to smoothly enter the fan module portion 140.

The air that enters the fan module portion 140 is discharged to the front of the fan module portion 140. The air discharged to the front of the fan module portion 140 may be discharged in a diagonal flow direction. Here, the diagonal flow direction may be defined as a forward diagonal direction.

Air suctioned into a central portion of the rear of the fan module portion 140 is moved to the front through a discharge port provided in an annular shape along an inner side of an edge of the fan module portion 140 and is moved to the front through a space formed between supports of a rotation support portion. That is, since a diagonal flow fan is applied to the fan module portion 140, the air that enters through the rear of the fan module portion 140 may be discharged in a direction that is inclined forward, and an air movement path of the rotation support portion may coincide with a flow path direction. Thus, flow loss of air may be reduced.

Also, the air discharged to the front of the fan module portion 140 is moved along a curved surface at an inner side of the first case portion 20, which is disposed between both side corners of the fan module portion 140 in the width direction W and both side corners of the inlet portion 32 in the width direction W, is guided to move in a straight line in the first direction, and is discharged to the front of the housing portion 10 through the discharge portion. Since the inner side of the first case portion 20 is formed in a curved shape, and the inner side of the first case portion 20 that comes in contact with the fan module portion 140 has an inner diameter in the width direction W that gradually decreases toward the inlet portion 32, the inner side of the first case portion 20 serves as an air guide that allows air that exits the fan module portion 140 to move toward the inlet portion 32. Here, since the inner side of the first case portion 20 forms a concave curved surface, friction due to coming in contact with air moving from the fan module portion 140 toward the inlet portion 32 is minimized such that air flow performance is improved, and an ability of air to move forward in a straight line may be maintained.

Meanwhile, since the inlet grilles 33 disposed at the inlet portion 32 and the outlet grilles 37 disposed at the outlet portion 36 extend in the first direction, air passing through portions between the inlet grilles 33 and air being discharged through the outlet grilles 37 may be moved in a straight line in the first direction. Thus, a movement path of air passing through the housing portion 10 is shortened, and an air blowing ability is improved.

### [Arrangement of components and spacing between components]

As illustrated in FIG. 11, the first horizontal center line passing through the center of the housing portion 10 in the transverse direction is located at the center of the inlet portion 32 and the outlet portion 36. The first horizontal center line is a virtual line that passes through a portion with the largest diameter of the transverse cross-section of the first case portion 20. Also, the second horizontal center line is perpendicular to the first horizontal center line and extends in the first direction.

The portable air caring apparatus 1 is formed in a substantially cylindrical shape and extends in the vertical direction. For the portable air caring apparatus 1 to be mounted in a cup holder of a vehicle, components for air caring, sanitizing, and disinfecting are installed in the portable air caring apparatus 1 within a set size. Therefore, the arrangement of the components is one aspect of interest of the present invention.

Since the arrangement of the fan module portion 140 and the filter portion 70 is an important factor in the portable air caring apparatus 1, with respect to the first horizontal center line, the filter portion 70 is disposed at the inlet portion 32 while the fan module portion 140 is disposed at the outlet portion 36. Therefore, since air, from which foreign substances are removed due to the air passing through the filter portion 70, passes through the fan module portion 140, an occurrence of a phenomenon in which foreign substances such as dust are stuck in the fan module portion 140 is reduced, and the time and cost for maintenance and repair may be reduced.

Also, the sanitizing portion 210 for disinfecting the filter portion 70 using the sanitizing light should be spaced a predetermined distance apart from the filter portion 70 due to an irradiation angle of the sanitizing light, the number of installed sanitizing light sources 212 and positions thereof, and the like. Therefore, the sanitizing portion 210 is disposed in a direction toward the fan module portion 140 with respect to the first horizontal center line. Since an air blowing function is significantly degraded when the installation space of the fan module portion 140 is reduced, it is difficult to reduce the installation space of the fan module portion 140, but the installation space may be reduced when a high functional filter is used as the filter portion 70.

Therefore, when a distance between the first horizontal center line P1 and the filter portion 70 is D1 and a distance between the first horizontal center line P1 and the fan module portion 140 is D2, D1 and D2 have different values. Also, preferably, D 1 may have a greater value than D2.

For example, when D1 and D2 are equal or D2 is greater than D1, the installation space of the fan module portion 140 is reduced as compared to when D1 is greater than D2, and the air blowing function is significantly degraded. Thus, there is a problem in that air cleaning efficiency is also degraded.

On the other hand, in the case in which D1 has a greater value than D2, since the installation space of the fan module portion 140 may be secured, degradation of the air blowing function is prevented, and thus there is an effect of preventing the degradation of air cleaning efficiency while allowing an operation of sanitizing the filter portion 70 to be performed. That is, since the air blowing ability of the fan module portion 140 is an important factor in an air caring function, the fan module portion 140 is installed to be closer to the first horizontal center line P1 than the filter portion 70. Thus, the volume of the fan module portion 140 is formed to be larger than the volume of the filter portion 70.

Also, the second horizontal center line that intersects the first horizontal center line at a right angle at the center of the housing portion 10 and extends in the transverse direction may pass through the center of the inlet portion 32, the filter portion 70, the sanitizing portion 210, the fan module portion 140, and the outlet portion 36 in the width direction W. Therefore, linearity of air sequentially passing through the inlet portion 32, the filter portion 70, the sanitizing portion 210, the fan module portion 140, and the outlet portion 36 is further improved, and the air blowing function is improved. Thus, the air cleaning efficiency is also improved.

Meanwhile, since the sanitizing portion 210 is fixed in a state of being inserted into the filter case 80, reduction of the length of the fan module portion 140 or the filter portion 70 in the first direction due to installing the sanitizing portion 210 may be minimized. Thus, degradation of the air blowing function may also be prevented.

Also, the rear of the sanitizing portion 210 is supported by the fixing protrusion 116, and the front of the sanitizing portion 210 is supported by the bell mouth 202 of the fan base 200 or supported by the bell mouth 202 and the locking protrusion 117. Therefore, a separate bracket for fixing the sanitizing portion 210 is not necessary, and a length of another component in the first direction is not reduced due to a separately provided bracket. Thus, an excellent air caring function may be provided, and due to the reduced number of components, the production costs and maintenance and repair costs may also be reduced.

In a portable air caring apparatus according to the present invention, since a sanitizing portion is installed between a filter portion and a fan module portion to sanitize the filter portion, contamination of air can be prevented.

Also, according to the present invention, since the sanitizing portion is installed between the filter portion and the fan module portion and since sanitizing light irradiated toward the filter portion, which blocks an inlet portion, is prevented from leaking to the outside of a housing portion, a safe usage environment can be provided.

Also, according to the present invention, since it is possible to fix the sanitizing portion between the filter case and the fan module portion or fix the sanitizing portion inside the filter case without a separate fixing bracket to fix the sanitizing portion, an increase in resistance of an air flow path can be minimized to improve air caring performance.

Also, according to the present invention, a separate fixing bracket for fixing the sanitizing portion can be added. In addition, according to the present invention, the fixing bracket for fixing the sanitizing portion can be installed at a position for minimizing an increase in resistance of an air flow path to improve air caring performance.

Also, according to the present invention, in a state in which the sanitizing portion is disposed at the center of the fan module portion in the width direction and extends in the vertical direction, an upper end and a lower end of the sanitizing portion do not protrude to the outside of a hub of fan members. Since an upper end and a lower end of the fan module portion do not face a suction space formed outside the hub, an increase in resistance of an air flow path along which air is suctioned into the fan member can be minimized to improve air caring performance.

Also, according to the present invention, since air that enters an inlet portion of the housing portion moves in the horizontal direction, sequentially passes through the filter portion, the sanitizing portion, and the fan module portion, and then is discharged through an outlet portion of the housing portion, heat dissipation of the sanitizing portion is easily performed and durability of the sanitizing portion is improved. Thus, a movement path of the air is shortened and air caring performance can be improved.

Also, according to the present invention, since the fan module portion is installed to be closer to a first horizontal center line than the filter portion, the filter portion is sanitized while degradation of air caring efficiency is minimized, and thus contamination of air can be prevented. In this way, user satisfaction can be improved.

Also, according to the present invention, since a second case portion is mounted on a structure such as a cup holder having the shape of a groove which is concave toward the lower side and since air can be suctioned through the inlet portion provided in the first case portion without interfering with an external structure, air caring performance can be improved.

Also, according to the present invention, since a transverse cross-section of the housing portion that is installed in a concave groove portion, such as a cup holder of a vehicle, is formed in a circular shape or an elliptical shape, as compared to a housing portion whose transverse cross-section is formed in a polygonal shape, a separation distance between the housing portion and the cup holder can be reduced, and thus an occurrence of vibration and noise can be reduced.

In addition to the above-described effects, specific effects of the present invention have been described above along with specific details for carrying out the invention.

The present invention has been described above with reference to the accompanying drawings, but the present invention is not limited by the embodiments disclosed herein and the drawings, and it is apparent that various modifications may be made by those of ordinary skill in the art to which the present invention pertains. Further, even when the effects according to configurations of the present invention are not explicitly described while describing the embodiments of the present invention, predictable effects of the corresponding configurations should also be recognized.

## Claims

1. A portable air caring apparatus (1) comprising:
a housing portion (10) which has an accommodation space (22) formed therein and includes an inlet portion (32) configured to suction air and an outlet portion (36) configured to discharge air;
a filter portion (70) installed at an inner side of the housing portion (10) that faces the inlet portion (32);
a fan module portion (140) installed at an inner side of the housing portion (10) that faces the outlet portion (36);
a filter case (80) in which the filter portion (70) is detachably installed and which is disposed between the filter portion (70) and the fan module portion (140) and configured to guide movement of air from the filter portion (70) to the fan module portion (140); and
a sanitizing portion (210) which is mounted on the filter case (80) and configured to irradiate sanitizing light toward the filter portion (70),
wherein air that enters through the inlet portion (32) is guided in a horizontal direction so that the air sequentially passes through the filter portion (70), the filter case (80), and the fan module portion (140) and then is discharged to the outside of the housing portion (10) through the outlet portion (36), and
wherein the filter case (80) includes:
a case main body portion (90) which is fixed to the inside of the housing portion (10) and in which the filter portion (70) is mounted and both longitudinal sides are open;
a flow path guiding portion (100) which is installed at one side of the case main body portion (90) and forms a path along which air moves from the filter portion (70) toward the fan module portion (140); and
a fixing supporting portion (110) which is disposed at the center of the flow path guiding portion (100), extends in the vertical direction, and includes a protrusion configured to support the sanitizing portion (210).

2. The portable air caring apparatus of claim 1, wherein the housing portion (10) includes:
a first case portion (20) in which the filter portion (70), the sanitizing portion (210), and the fan module portion (140) are installed, wherein the inlet portion (32) is disposed at one side surface, and the outlet portion (36) configured to discharge air is disposed at the other side surface opposite the inlet portion (32); and
a second case portion (60) connected to a lower portion of the first case portion (20).

3. The portable air caring apparatus of claim 2, wherein an air flow path is formed inside the first case portion (20), and an air flow path is not formed inside the second case portion (60).

4. The portable air caring apparatus of claim 2 or 3, wherein a transverse cross-section of at least any one of the first case portion (20) and the second case portion (60) is formed in a circular shape or an elliptical shape.

5. The portable air caring apparatus of any one of claims 2 to 4, wherein the first case portion (20) includes:
a first case body (30) which includes the inlet portion (32) and the outlet portion (36) and has a service space provided at a side surface; and
a service door (50) which is detachably installed at the first case body (30) to open and close the service space.

6. The portable air caring apparatus of claim 5, wherein the service door includes:
a door body (52) detachably installed at the first case body (30);
a gap maintaining protrusion (54) which protrudes to the inside of the door body (52) and extends toward at least any one of the filter portion (70) and the filter case (80); and
a first mounting groove portion (56) which forms a groove inside the door body (52) in a vertical direction,
wherein optionally the first case body (30) further includes a second mounting groove portion (40) which forms a groove at an inner side of the first case body (30), which faces the first mounting groove portion (56), in the vertical direction, and
one side and the other side of the fan module portion (140) in a width direction are inserted into the first mounting groove portion (56) and the second mounting groove portion (40) to restrict movement of the fan module portion (140).

7. The portable air caring apparatus of any one of claims 1 to 6, wherein:
in the inlet portion (32), a plurality of inlet grilles (33) that extend in the vertical direction are installed at one side surface of the housing portion (10) to form inlet holes (34); and
in the outlet portion (36), a plurality of outlet grilles (37) that extend in the vertical direction are installed at the other side surface of the housing portion (10) to form outlet holes (38).

8. The portable air caring apparatus of any one of claims 1 to 7, wherein the filter portion (70) is disposed at an inner side of the housing portion (10) and faces the inlet portion (32), and
both sides of the filter portion (70) in the width direction are installed in a state of coming in contact with the inner side of the housing portion (10),
wherein optionally the filter portion (70) is formed in a rectangular parallelepiped shape extending in the vertical direction and is mounted inside the filter case (80) such that movement of the filter portion (70) is restricted.

9. The portable air caring apparatus of any one of claims 1 to 8, wherein:
the filter case (80) further includes a catching step (120) which protrudes to the inside of the case main body portion (90) and restricts movement of the filter portion (70), and
the filter portion (70) caught on the catching step (120) is installed to be spaced a predetermined distance apart from the sanitizing portion (210), and/or
the filter case (80) further includes:
a first fixing protrusion (130) which protrudes to an upper side of the case main body portion (90) and is fixed to a first boss (42) disposed at an inner side of the housing portion (10); and
a second fixing protrusion (132) which protrudes to a lower side of the case main body portion (90) and is fixed to a second boss (44) disposed at an inner side of the housing portion (10), and/or
the filter case (80) further includes a first coupling protrusion (134) which protrudes in a direction toward the fan module portion (140) and is inserted into the fan module portion (140), and/or
flow path guiding portion (100) is installed at both sides of the fixing supporting portion (110), extends in the vertical direction, and includes a plurality of through-holes (102), and/or
the fixing supporting portion (110) includes:
a filter frame (112) which forms an inner hole (114) extending in the vertical direction and is connected to the flow path guiding portion (100); and
a plurality of fixing protrusions (116) which protrude from the filter frame (112) toward the inner hole (114),
wherein the sanitizing portion (210) is inserted into the filter frame (112), and movement of the sanitizing portion (210) is restricted due to the sanitizing portion (210) being caught on the fixing protrusion (116).

10. The portable air caring apparatus of any one of claims 1 to 9, wherein the sanitizing portion (210) is installed at the center of the filter case (80) in the width direction and extends in the vertical direction.

11. The portable air caring apparatus of claim 10, wherein:
the sanitizing portion (210) includes:
a printed circuit board (211) which extends in the vertical direction; and
a sanitizing light source (212) which is installed at both sides of the printed circuit board (211) in the vertical direction and is configured to irradiate sanitizing light toward the filter portion (70), and/or
the sanitizing portion (210) is disposed at the center of the fan module portion (140) in the width direction, and two fan members (160) are installed in the vertical direction along the sanitizing portion (210); an upper end of the sanitizing portion (210) is installed at a position lower than a position of an upper end of a hub (170) of the fan member (160) that is disposed at the upper side of the sanitizing portion (210); and
a lower end of the sanitizing portion (210) is installed at a position higher than a position of a lower end of the hub (170) of the fan member (160) that is disposed at the lower side of the sanitizing portion (210).

12. The portable air caring apparatus of any one of claims 1 to 11, wherein the fan module portion (140) includes:
a fan housing (150) which is fixed to an inner side of the housing portion (10);
a fan member (160) which is rotatably installed inside the fan housing (150) and configured to move air in a direction toward the outlet portion (36); and
a fan base (200) which is coupled to the fan housing (150) and configured to guide air, which has passed through the filter portion (70), to enter the fan member (160).

13. The portable air caring apparatus of claim 12, wherein:
as the fan housing (150) and the fan member (160), two fan housings (150) and two fan members (160) are installed in the vertical direction of the fan base (200), and a central axis of rotation of the fan member (160) intersects the sanitizing portion (210) at a right angle, and/or
the fan housing (150) includes:
a support plate (151) which is installed in the shape of a plate at a position facing the fan member (160);
a connection support (152) which extends toward the outside of the support plate (151);
a side support portion (154) which is connected to the connection support (152) and forms a circular or quadrilateral frame around the periphery of the support plate (151); and
a protruding boss (156) which protrudes in a direction from the side support portion (154) toward the fan base (200) and is coupled to the fan base (200).

14. The portable air caring apparatus of claim 12 or 13, wherein the fan member (160) includes:
a hub (170) which is disposed at the center of the fan housing (150) and configured to receive external power and rotate;
a plurality of fan blades (180) disposed to be spaced apart from each other at equal intervals along an outer peripheral surface of the hub (170); and
a shroud (190) which is connected to an end portion of the fan blade (180), installed in an annular shape, and spaced apart from the fan base (200),
wherein optionally the fan base (200) includes:
a base plate (201) which is formed in the shape of a plate that includes a hollow for air movement and extends in the vertical direction;
a bell mouth (202) which is installed in an annular shape at an inner side of the base plate (201) that faces the hollow and which includes a longitudinal end surface that has a concave shape surrounding a lower side of an inlet protrusion (191) of the shroud (190); and
a coupling protrusion (204) which protrudes from the base plate (201) and is coupled to the protruding boss (156) of the fan housing (150).

15. The portable air caring apparatus of any one of claims 1 to 14,
wherein a first horizontal center line (P1) passing through the center of the housing portion (10) in the transverse direction is disposed at the center of the inlet portion (32) and the outlet portion (36), and
when a distance between the first horizontal center line (P1) and the filter portion (70) is D1 and a distance between the first horizontal center line (P1) and the fan module portion (140) is D2, D1 has a greater value than D2.

16. The portable air caring apparatus of claim 15, wherein:
the center of the inlet portion (32) in the width direction, a center of the filter portion (70) in the width direction, a center of the sanitizing portion (210) in the width direction, a center of the fan module portion (140) in the width direction, and the center of the outlet portion (36) in the width direction coincide with each other, and/or
a second horizontal center line (P2) that intersects the first horizontal center line (P1) at a right angle at the center of the housing portion (10) and extends in the transverse direction passes through the center of the inlet portion (32), the filter portion (70), the sanitizing portion (210), the fan module portion (140), and the outlet portion (36) in the width direction, and/or

17. The portable air caring apparatus of claim 15 or 16, wherein:
a plurality of fan members (160) are installed in the vertical direction in the fan module portion (140); and
the sanitizing portion (210) is installed at a position parallel to a vertical virtual line (V) that perpendicularly intersects the central axis of rotation of the fan members (160) and extends in the vertical direction, and/or
one side surface of the sanitizing portion (210) is supported by a fixing protrusion (116) of the filter case (80); and
the other side surface of the sanitizing portion (210) is supported by the fan module portion (140).

## Patentansprüche

1. Tragbares Luftreinigungsgerät (1), aufweisend:
einen Aufnahmeabschnitt (10), in dem ein Aufnahmeraum (22) ausgebildet ist und der einen Einlassabschnitt (32) zum Ansaugen von Luft und einen Auslassabschnitt (36) zum Ausgeben von Luft aufweist;
einen Filterabschnitt (70), der an einer dem Einlassabschnitt (32) zugewandten Innenseite des Aufnahmeabschnitts (10) angebracht ist;
einen Lüftermodulabschnitt (140), der an einer dem Auslassabschnitt (36) zugewandten Innenseite des Aufnahmeabschnitts (10) angebracht ist;
ein Filtergehäuse (80), in dem der Filterabschnitt (70) abnehmbar angebracht ist und das zwischen dem Filterabschnitt (70) und dem Lüftermodulabschnitt (140) angeordnet und dazu ausgelegt ist, die Luftbewegung von dem Filterabschnitt (70) zu dem Lüftermodulabschnitt (140) zu führen; und
einen Desinfektionsabschnitt (210), der an dem Filtergehäuse (80) gelagert und dazu ausgelegt ist, desinfizierendes Licht in Richtung des Filterabschnitts (70) auszustrahlen,
wobei Luft, die durch den Einlassabschnitt (32) eintritt, in einer horizontalen Richtung geführt wird, so dass die Luft nacheinander durch den Filterabschnitt (70), das Filtergehäuse (80) und den Lüftermodulabschnitt (140) strömt und dann durch den Auslassabschnitt (36) zur Außenseite des Aufnahmeabschnitts (10) ausgegeben wird, und
wobei das Filtergehäuse (80) aufweist:
einen Gehäusehauptkörperabschnitt (90), der an der Innenseite des Aufnahmeabschnitts (10) befestigt ist und in dem der Filterabschnitt (70) gelagert ist und von dem beide Längsseiten offen sind;
einen Strömungspfadführungsabschnitt (100), der an einer Seite des Gehäusehauptkörperabschnitts (90) angebracht ist und einen Pfad bildet, entlang dessen sich Luft von dem Filterabschnitt (70) zu dem Lüftermodulabschnitt (140) bewegt; und
einen Befestigungslagerabschnitt (110), der im Zentrum des Strömungspfadführungsabschnitts (100) angeordnet ist, sich in vertikaler Richtung erstreckt und einen Vorsprung zum Lagern des Desinfektionsabschnitts (210) aufweist.

2. Tragbares Luftreinigungsgerät nach Anspruch 1, wobei der Aufnahmeabschnitt (10) aufweist:
einen ersten Gehäuseabschnitt (20), in dem der Filterabschnitt (70), der Desinfektionsabschnitt (210) und der Lüftermodulabschnitt (140) angebracht sind, wobei der Einlassabschnitt (32) an einer Seitenfläche angeordnet ist und der Auslassabschnitt (36) zum Ausgeben von Luft an der anderen, dem Einlassabschnitt gegenüberliegenden Seitenfläche (32) angeordnet ist; und
einen zweiten Gehäuseabschnitt (60), der mit einem unteren Bereich des ersten Gehäuseabschnitts (20) verbunden ist.

3. Tragbares Luftreinigungsgerät nach Anspruch 2, wobei innerhalb des ersten Gehäuseabschnitts (20) ein Luftströmungspfad ausgebildet ist und innerhalb des zweiten Gehäuseabschnitts (60) kein Luftströmungspfad ausgebildet ist.

4. Tragbares Luftreinigungsgerät nach Anspruch 2 oder 3, wobei ein Querschnitt des ersten Gehäuseabschnitts (20) und/oder des zweiten Gehäuseabschnitts (60) kreisförmig oder elliptisch ausgebildet ist.

5. Tragbares Luftreinigungsgerät nach einem der Ansprüche 2 bis 4, wobei der erste Gehäuseabschnitt (20) aufweist:
einen ersten Gehäusekörper (30), der den Einlassabschnitt (32) und den Auslassabschnitt (36) aufweist und einen an einer Seitenfläche vorgesehenen Wartungsraum aufweist; und
eine Wartungstür (50), die abnehmbar an dem ersten Gehäusekörper (30) angebracht ist, um den Wartungsraum zu öffnen und zu schließen.

6. Tragbares Luftreinigungsgerät nach Anspruch 5, wobei die Wartungstür aufweist:
einen Türkörper (52), der abnehmbar an dem ersten Gehäusekörper (30) angebracht ist;
einen Spalthaltevorsprung (54), der zum Inneren des Türkörpers (52) ragt und sich in Richtung des Filterabschnitts (70) und/oder des Filtergehäuses (80) erstreckt; und
einen ersten Montagenutabschnitt (56), der im Inneren des Türkörpers (52) eine Nut in einer vertikalen Richtung bildet,
wobei optional der erste Gehäusekörper (30) ferner einen zweiten Montagenutabschnitt (40) aufweist, der an einer dem ersten Montagenutabschnitt (56) zugewandten Innenseite des ersten Gehäusekörpers (30) eine Nut in der vertikalen Richtung bildet, und
wobei die eine Seite und die andere Seite des Lüftermodulabschnitts (140) in einer Breitenrichtung in den ersten Montagenutabschnitt (56) und den zweiten Montagenutabschnitt (40) eingesetzt sind, um die Bewegung des Lüftermodulabschnitts (140) einzuschränken.

7. Tragbares Luftreinigungsgerät nach einem der Ansprüche 1 bis 6, wobei
im Einlassabschnitt (32mehrere in vertikaler Richtung verlaufende Einlassgitter (33) an einer Seitenfläche des Aufnahmeabschnitts (10) angebracht sind, um Einlasslochungen (34) zu bilden; und
in dem Auslassabschnitt (36) mehrere in vertikaler Richtung verlaufende Auslassgitter (37) an der anderen Seitenfläche des Aufnahmeabschnitts (10) angebracht sind, um Auslasslochungen (38) zu bilden.

8. Tragbares Luftreinigungsgerät nach einem der Ansprüche 1 bis 7, wobei der Filterabschnitt (70) an einer Innenseite des Aufnahmeabschnitts (10) angeordnet und dem Einlassabschnitt (32) zugewandt ist, und
beide Seiten des Filterabschnitts (70) in der Breitenrichtung so angebracht sind, dass sie mit der Innenseite des Aufnahmeabschnitts (10) in Kontakt gelangen,
wobei optional der Filterabschnitt (70) in einer in vertikaler Richtung verlaufenden rechteckigen Quaderform ausgebildet ist und im Inneren des Filtergehäuses (80) so gelagert ist, dass die Bewegung des Filterabschnitts (70) eingeschränkt ist.

9. Tragbares Luftreinigungsgerät nach einem der Ansprüche 1 bis 8, wobei
das Filtergehäuse (80) ferner eine Fangstufe (120) aufweist, die zum Inneren des Gehäusehauptkörperabschnitts (90) ragt und die Bewegung des Filterabschnitts (70) einschränkt, und
der Filterabschnitt (70), der an der Fangstufe (120) gefangen ist, so angebracht ist, dass er in einem vorbestimmten Abstand von dem Desinfektionsabschnitt (210) entfernt liegt, und/oder
das Filtergehäuse (80) ferner aufweist:
einen ersten Befestigungsvorsprung (130), der zu einer Oberseite des Gehäusehauptkörperabschnitts (90) abragt und an einer ersten Erhebung (42) befestigt ist, die an einer Innenseite des Aufnahmeabschnitts (10) angeordnet ist; und
einen zweiten Befestigungsvorsprung (132), der zu einer Unterseite des Gehäusehauptkörperabschnitts (90) abragt und an einer zweiten Erhebung (44) befestigt ist, die an einer Innenseite des Aufnahmeabschnitts (10) angeordnet ist, und/oder
das Filtergehäuse (80) ferner einen ersten Kopplungsvorsprung (134) aufweist, der in Richtung zum Lüftermodulabschnitt (140) hin abragt und in den Lüftermodulabschnitt (140) eingesetzt ist, und/oder
wobei ein Strömungspfadführungsabschnitt (100) an beiden Seiten des Befestigungslagerabschnitts (110) angebracht ist, sich in der vertikalen Richtung erstreckt und mehrere Durchgangslochungen (102) aufweist, und/oder
der Befestigungslagerabschnitt (110) aufweist:
einen Filterrahmen (112), der eine in vertikaler Richtung verlaufende innere Lochung (114) bildet und mit dem Strömungspfadführungsabschnitt (100) verbunden ist; und
mehrere Befestigungsvorsprünge (116), die von dem Filterrahmen (112) in Richtung der inneren Lochung (114) abragen,
wobei der Desinfektionsabschnitt (210) in den Filterrahmen (112) eingesetzt ist und die Bewegung des Desinfektionsabschnitts (210) dadurch eingeschränkt ist, dass der Desinfektionsabschnitt (210) an dem Befestigungsvorsprung (116) gefangen ist.

10. Tragbares Luftreinigungsgerät nach einem der Ansprüche 1 bis 9, wobei der Desinfektionsabschnitt (210) im Zentrum des Filtergehäuses (80) in Breitenrichtung angebracht ist und in vertikaler Richtung verläuft.

11. Tragbares Luftreinigungsgerät nach Anspruch 10, wobei
der Desinfektionsabschnitt (210) aufweist:
eine gedruckte Leiterplatte (211), die in vertikaler Richtung verläuft; und
eine Desinfektionslichtquelle (212), die an beiden Seiten der gedruckten Leiterplatte (211) in vertikaler Richtung angebracht und dazu ausgelegt ist, Desinfektionslicht in Richtung des Filterabschnitts (70) abzustrahlen, und/oder
der Desinfektionsabschnitt (210) im Zentrum des Lüftermodulabschnitts (140) in Breitenrichtung angeordnet ist und zwei Lüfterelemente (160) in der vertikalen Richtung entlang des Desinfektionsabschnitts (210) angebracht sind; wobei ein oberes Ende des Desinfektionsabschnitts (210) an einer Position angebracht ist, die niedriger ist als eine Position eines oberen Endes einer Nabe (170) des Lüfterelements (160), das an der Oberseite des Desinfektionsabschnitts (210) angeordnet ist; und
ein unteres Ende des Desinfektionsabschnitts (210) an einer Position angebracht ist, die höher ist als eine Position eines unteren Endes der Nabe (170) des Lüfterelements (160), das an der Unterseite des Desinfektionsabschnitts (210) angeordnet ist.

12. Tragbares Luftreinigungsgerät nach einem der Ansprüche 1 bis 11, wobei das Lüftermodulabschnitt (140) aufweist:
ein Lüftergehäuse (150), das an einer Innenseite des Aufnahmeabschnitts (10) befestigt ist;
ein Lüfterelement (160), das drehbar im Inneren des Lüftergehäuses (150) angebracht und dazu ausgelegt ist, Luft in eine Richtung zum Auslassabschnitt (36) zu bewegen; und
eine Lüfterbasis (200), die mit dem Lüftergehäuse (150) gekoppelt und dazu ausgelegt ist, Luft, die den Filterabschnitt (70) passiert hat, zu führen, um in das Lüfterelement (160) einzuströmen.

13. Tragbares Luftreinigungsgerät nach Anspruch 12, wobei
als Lüftergehäuse (150) und Lüfterelement (160) zwei Lüftergehäuse (150) und zwei Lüfterelemente (160) in der vertikalen Richtung der Lüfterbasis (200) angebracht sind und eine zentrale Drehachse des Lüfterelements (160) den Desinfektionsabschnitt (210) in einem rechten Winkel schneidet, und/oder
das Lüftergehäuse (150) aufweist:
eine Tragplatte (151), die in Form einer Platte an einer dem Lüfterelement (160) zugewandten Position angebracht ist;
einen Verbindungsträger (152), der sich in Richtung der Außenseite der Tragplatte (151) erstreckt;
einen seitlichen Tragabschnitt (154), der mit dem Verbindungsträger (152) verbunden ist und einen kreisförmigen oder viereckigen Rahmen um den Umfang der Tragplatte (151) bildet; und
eine vorspringende Erhebung (156), die in einer Richtung von dem seitlichen Tragabschnitt (154) zu der Lüfterbasis (200) hin vorspringt und mit der Lüfterbasis (200) gekoppelt ist.

14. Tragbares Luftreinigungsgerät nach Anspruch 12 oder 13, wobei das Lüfterelement (160) aufweist:
eine Nabe (170), die im Zentrum des Lüftergehäuses (150) angeordnet und dazu ausgelegt ist, Energie von außen aufzunehmen und sich zu drehen;
mehrere Lüfterflügel (180), die so angeordnet sind, dass sie in gleichen Abständen entlang einer äußeren Umfangsfläche der Nabe (170) voneinander beabstandet sind; und
eine Abdeckung (190), die mit einem Endabschnitt des Lüfterflügels (180) verbunden, in ringförmiger Gestalt angebracht und von der Lüfterbasis (200) beabstandet ist,
wobei optional die Lüfterbasis (200) aufweist:
eine Basisplatte (201), die in Form einer Platte ausgebildet ist, die einen Hohlraum für die Luftbewegung aufweist und in vertikaler Richtung verläuft;
eine Aufweitung (202), die in ringförmiger Gestalt an einer dem Hohlraum zugewandten Innenseite der Basisplatte (201) angebracht ist und eine Längsendfläche mit einer konkaven Form aufweist, die eine Unterseite eines Einlassvorsprungs (191) der Abdeckung (190) umgibt; und
einen Kopplungsvorsprung (204), der von der Basisplatte (201) abragt und mit der vorspringenden Erhebung (156) des Lüftergehäuses (150) gekoppelt ist.

15. Tragbares Luftreinigungsgerät nach einem der Ansprüche 1 bis 14,
wobei eine erste horizontale Mittellinie (P1), die durch das Zentrum des Aufnahmeabschnitts (10) in Querrichtung verläuft, im Zentrum des Einlassabschnitts (32) und des Auslassabschnitts (36) angeordnet ist, und
wobei, wenn ein Abstand zwischen der ersten horizontalen Mittellinie (P1) und dem Filterabschnitt (70) D1 ist und ein Abstand zwischen der ersten horizontalen Mittellinie (P1) und dem Lüftermodulabschnitt (140) D2 ist, D1 einen größeren Wert hat als D2.

16. Tragbares Luftreinigungsgerät nach Anspruch 15, wobei
das Zentrum des Einlassabschnitts (32) in Breitenrichtung, ein Zentrum des Filterabschnitts (70) in Breitenrichtung, ein Zentrum des Desinfektionsabschnitts (210) in Breitenrichtung, ein Zentrum des Lüftermodulabschnitts (140) in Breitenrichtung und das Zentrum des Auslassabschnitts (36) in Breitenrichtung miteinander übereinstimmen, und/oder
eine zweite horizontale Mittellinie (P2), die die erste horizontale Mittellinie (P1) in einem rechten Winkel im Zentrum Mitte des Aufnahmeabschnitts (10) schneidet und in Querrichtung verläuft, durch das Zentrum des Einlassabschnitts (32), des Filterabschnitts (70), des Desinfektionsabschnitts (210), des Lüftermodulabschnitts (140) und des Auslassabschnitts (36) in Breitenrichtung verläuft.

17. Tragbares Luftreinigungsgerät nach Anspruch 15 oder 16, wobei
mehrere Lüfterelemente (160) in vertikaler Richtung in dem Lüftermodulabschnitt (140) angebracht sind; und
der Desinfektionsabschnitt (210) an einer Position parallel zu einer vertikalen virtuellen Linie (V) angebracht ist, die die zentrale Drehachse der Lüfterelemente (160) senkrecht schneidet und in vertikaler Richtung verläuft, und/oder
eine Seitenfläche des Desinfektionsabschnitts (210) von einem Befestigungsvorsprung (116) des Filtergehäuses (80) getragen wird; und
die andere Seitenfläche des Desinfektionsabschnitts (210) von dem Lüftermodulabschnitt (140) getragen wird.

## Revendications

1. Appareil de traitement de l'air portable (1) comprenant :
une partie de boîtier (10) qui présente un espace de logement (22) formé à l'intérieur et comporte une partie d'entrée (32) configurée pour aspirer de l'air et une partie de sortie (36) configurée pour évacuer de l'air ;
une partie de filtre (70) installée sur un côté intérieur de la partie de boîtier (10) qui fait face à la partie d'entrée (32) ;
une partie de module de ventilateur (140) installée sur un côté intérieur de la partie de boîtier (10) qui fait face à la partie de sortie (36) ;
un carter de filtre (80) dans lequel la partie de filtre (70) est installée de manière amovible et qui est disposé entre la partie de filtre (70) et la partie de module de ventilateur (140) et est configuré pour guider le mouvement de l'air de la partie de filtre (70) à la partie de module de ventilateur (140) ; et
une partie d'assainissement (210) qui est montée sur le carter de filtre (80) et est configurée pour irradier la lumière d'assainissement vers la partie de filtre (70),
dans lequel de l'air qui entre à travers la partie d'entrée (32) est guidé dans une direction horizontale de telle sorte que l'air passe de manière séquentielle à travers la partie de filtre (70), le carter de filtre (80) et la partie de module de ventilateur (140) et est ensuite évacué vers l'extérieur de la partie de boîtier (10) à travers la partie de sortie (36), et
dans lequel le carter de filtre (80) comporte :
une partie de corps principale de carter (90), qui est fixée à l'intérieur de la partie de boîtier (10) et dans laquelle la partie de filtre (70) est montée et les deux côtés longitudinaux sont ouverts ;
une partie de guidage de trajet d'écoulement (100) qui est installée sur un côté de la partie de corps principale de carter (90) et forme un trajet, le long duquel de l'air se déplace depuis la partie de filtre (70) vers la partie de module de ventilateur (140) ; et
une partie de support de fixation (110), qui est disposée au centre de la partie de guidage de trajet d'écoulement (100), s'étend dans la direction verticale et comporte une partie faisant saillie configurée pour supporter la partie d'assainissement (210).

2. Appareil portable de traitement de l'air selon la revendication 1, dans lequel la partie de boîtier (10) comporte :
une première partie de boîtier (20) dans laquelle la partie de filtre (70), la partie d'assainissement (210) et la partie de module de ventilateur (140) sont installées, dans lequel la partie d'entrée (32) est disposée sur une surface latérale, et la partie de sortie (36) configurée pour évacuer l'air est disposée sur l'autre surface latérale opposée à la partie d'entrée (32) ; et
une deuxième partie de carter (60) reliée à une partie inférieure de la première partie de carter (20).

3. Appareil portable de traitement de l'air selon la revendication 2, dans lequel un trajet d'écoulement d'air est formé à l'intérieur de la première partie de carter (20), et un trajet d'écoulement d'air n'est pas formé à l'intérieur de la deuxième partie de carter (60).

4. Appareil portable de traitement de l'air selon la revendication 2 ou 3, dans lequel une section transversale d'au moins l'une quelconque de la première partie de carter (20) et de la deuxième partie de carter (60) est formée en une forme circulaire ou une forme elliptique.

5. Appareil portable de traitement de l'air selon l'une quelconque des revendications 2 à 4, dans lequel la première partie de carter (20) comporte :
un premier corps de carter (30) qui comporte la partie d'entrée (32) et la partie de sortie (36) et présente un espace de service prévu sur une surface latérale ; et
une porte de service (50) qui est installée de manière amovible sur le premier corps de carter (30) pour ouvrir et fermer l'espace de service.

6. Appareil portable de traitement de l'air selon la revendication 5, dans lequel la porte de service comporte :
un corps de porte (52) installé de manière amovible sur le premier corps de carter (30) ;
une partie faisant saillie maintenant l'espace (54) qui fait saillie vers l'intérieur du corps de porte (52) et s'étend vers au moins un de la partie de filtre (70) et du carter de filtre (80) ; et
une première partie de rainure de montage (56) qui forme une rainure à l'intérieur du corps de porte (52) dans une direction verticale,
dans lequel, en option, le premier corps de carter (30) comporte en outre une deuxième partie de rainure de montage (40) qui forme une rainure sur un côté intérieur du premier corps de carter (30), qui fait face à la première partie de rainure de montage (56) dans la direction verticale, et
un côté et l'autre côté de la partie de module de ventilateur (140) dans une direction de largeur sont insérés dans la première partie de rainure de montage (56) et la deuxième partie de rainure de montage (40) pour limiter le mouvement de la partie de module de ventilateur (140).

7. Appareil portable de traitement de l'air selon l'une quelconque des revendications 1 à 6, dans lequel :
dans la partie d'entrée (32), une pluralité de grilles d'entrée (33) qui s'étendent dans la direction verticale sont installées sur une surface latérale de la partie de boîtier (10) pour former des trous d'entrée (34) ; et
dans la partie de sortie (36), une pluralité de grilles de sortie (37) qui s'étendent dans la direction verticale sont installées sur l'autre surface latérale de la partie de boîtier (10) pour former des trous de sortie (38).

8. Appareil portable de traitement de l'air selon l'une quelconque des revendications 1 à 7, dans lequel la partie filtre (70) est disposée sur un côté intérieur de la partie de boîtier (10) et fait face à la partie d'entrée (32), et
les deux côtés de la partie de filtre (70) dans la direction de la largeur sont installés dans un état dans lequel ils viennent en contact avec le côté intérieur de la partie de boîtier (10),
dans lequel, en option, la partie de filtre (70) est formée en une forme de parallélépipède rectangulaire s'étendant dans la direction verticale et est montée à l'intérieur du carter de filtre (80) de sorte que le mouvement de la partie de filtre (70) est limité.

9. Appareil portable de traitement de l'air selon l'une quelconque des revendications 1 à 8, dans lequel
le carter de filtre (80) comporte en outre un épaulement d'accrochage (120) qui fait saillie vers l'intérieur de la partie de corps principale de carter (90) et limite le mouvement de la partie de filtre (70), et
la partie de filtre (70) accrochée sur l'épaulement d'accrochage (120) est installée pour être espacée d'une distance prédéterminée de la partie d'assainissement (210), et/ou
le carter de filtre (80) comporte en outre :
- une première partie faisant saillie de fixation (130) qui fait saillie vers un côté supérieur de la partie de corps principale de carter (90) et est fixée à un premier bossage (42) disposé sur un côté intérieur de la partie de boîtier (10) ; et
- une deuxième partie faisant saillie de fixation (132) qui fait saillie vers un côté inférieur de la partie de corps principale de carter (90) et est fixée à un deuxième bossage (44) disposé sur un côté intérieur de la partie de boîtier (10), et/ou
le carter de filtre (80) comporte en outre une première partie faisant saillie de couplage (134) qui fait saillie dans une direction vers la partie de module de ventilateur (140) et est insérée dans la partie de module de ventilateur (140), et/ou
la partie de guidage de trajet d'écoulement (100) est installée sur les deux côtés de la partie de support de fixation (110), s'étend dans la direction verticale, et comporte une pluralité de trous traversants (102), et/ou
la partie de support de fixation (110) comporte :
- un cadre de filtre (112) qui forme un trou intérieur (114) s'étendant dans la direction verticale et est relié à la partie de guidage de trajet d'écoulement (100) ; et
- une pluralité de parties faisant saillie de fixation (116) qui font saillie depuis le cadre de filtre (112) vers le trou intérieur (114),
dans lequel la partie d'assainissement (210) est insérée dans le cadre de filtre (112), et le mouvement de la partie d'assainissement (210) est limité du fait que la partie d'assainissement (210) est prise sur la partie faisant saillie de fixation (116).

10. Appareil portable de traitement de l'air selon l'une quelconque des revendications 1 à 9, dans lequel la partie d'assainissement (210) est installée au centre du carter de filtre (80) dans la direction de la largeur et s'étend dans la direction verticale.

11. Appareil portable de traitement de l'air selon la revendication 10, dans lequel :
la partie d'assainissement (210) comporte :
- une carte de circuit imprimé (211) qui s'étend dans la direction verticale ; et
- une source de lumière d'assainissement (212) qui est installée des deux côtés de la carte de circuit imprimé (211) dans la direction verticale et est configurée pour irradier la lumière d'assainissement vers la partie de filtre (70), et/ou
la partie d'assainissement (210) est disposée au centre de la partie de module de ventilateur (140) dans la direction de la largeur, et deux éléments de ventilateur (160) sont installés dans la direction verticale le long de la partie d'assainissement (210) ; une extrémité supérieure de la partie d'assainissement (210) est installée sur une position inférieure à une position d'une extrémité supérieure d'un moyeu (170) de l'élément de ventilateur (160) qui est disposé sur le côté supérieur de la partie d'assainissement (210) ; et
une extrémité inférieure de la partie d'assainissement (210) est installée à une position plus haute qu'une position d'une extrémité inférieure du moyeu (170) de l'élément de ventilateur (160) qui est disposé sur le côté inférieur de la partie d'assainissement (210).

12. Appareil portable de traitement de l'air selon l'une quelconque des revendications 1 à 11, dans lequel la partie de module de ventilateur (140) comporte :
un boîtier de ventilateur (150) qui est fixé sur un côté intérieur de la partie de boîtier (10) ;
un élément de ventilateur (160) qui est installé de manière rotative à l'intérieur du boîtier de ventilateur (150) et est configuré pour déplacer de l'air dans une direction vers la partie de sortie (36) ; et
une base de ventilateur (200) qui est couplée au boîtier de ventilateur (150) et est configurée pour guider de l'air, qui est passé à travers la partie de filtre (70), pour entrer dans l'élément de ventilateur (160).

13. Appareil portable de traitement de l'air selon la revendication 12, dans lequel :
lorsque le boîtier de ventilateur (150) et l'élément de ventilateur (160), deux boîtiers de ventilateur (150) et deux éléments de ventilateur (160) sont installés dans la direction verticale de la base de ventilateur (200), et un axe central de rotation de l'élément de ventilateur (160) croise la partie d'assainissement (210) à angle droit, et/ou
le boîtier de ventilateur (150) comporte :
une plaque de support (151) qui est installée sous la forme d'une plaque sur une position faisant face à l'élément de ventilateur (160) ;
un support de liaison (152) qui s'étend vers l'extérieur de la plaque de support (151);
une partie de support latérale (154) qui est reliée au support de liaison (152) et forme un cadre circulaire ou quadrilatéral autour de la périphérie de la plaque de support (151) ; et
un bossage faisant saillie (156) qui fait saillie dans une direction depuis la partie de support latérale (154) vers la base de ventilateur (200) et est couplé à la base de ventilateur (200).

14. Appareil portable de traitement de l'air selon la revendication 12 ou 13, dans lequel l'élément ventilateur (160) comporte :
un moyeu (170) qui est disposé au centre du carter de ventilateur (150) et est configuré pour recevoir une puissance externe et tourner ;
une pluralité de pales de ventilateur (180) disposées de manière à être espacées les unes des autres à des intervalles égaux le long d'une surface périphérique extérieure du moyeu (170) ; et
un carénage (190) qui est raccordé à une partie d'extrémité de la pale de ventilateur (180), installé dans une forme annulaire, et espacé de la base de ventilateur (200),
dans lequel en option la base de ventilateur (200) comporte :
une plaque de base (201) qui est formée sous la forme d'une plaque qui comprend un creux pour le mouvement d'air et s'étend dans la direction verticale ;
un pavillon (202) qui est installé en une forme annulaire sur un côté intérieur de la plaque de base (201) qui fait face au creux et qui comporte une surface d'extrémité longitudinale qui présente une forme concave entourant un côté inférieur d'une partie faisant saillie d'entrée (191) du carénage (190) ; et
une partie faisant saillie de couplage (204) qui fait saillie depuis la plaque de base (201) et est couplée au bossage faisant saillie (156) du boîtier de ventilateur (150).

15. Appareil portable de traitement de l'air selon l'une quelconque des revendications 1 à 14,
dans lequel une première ligne centrale horizontale (P1) passant par le centre de la partie de boîtier (10) dans la direction transversale est disposée au centre de la partie d'entrée (32) et de la partie de sortie (36), et
lorsqu'une distance entre la première ligne centrale horizontale (P1) et la partie de filtre (70) est D1 et que la distance entre la première ligne centrale horizontale (P1) et la partie de module de ventilateur (140) est D2, D1 présente une valeur supérieure à D2.

16. Appareil portable de traitement de l'air selon la revendication 15, dans lequel :
le centre de la partie d'entrée (32) dans la direction de la largeur, un centre de la partie de filtre (70) dans la direction de la largeur, un centre de la partie d'assainissement (210) dans la direction de la largeur, un centre de la partie de module de ventilateur (140) dans la direction de la largeur, et le centre de la partie de sortie (36) dans la direction de la largeur coïncident les uns avec les autres, et/ou
une deuxième ligne centrale horizontale (P2) qui coupe la première ligne centrale horizontale (P1) à angle droit au centre de la partie de boîtier (10) et s'étend dans la direction transversale passe par le centre de la partie d'entrée (32), de la partie de filtre (70), de la partie d'assainissement (210), de la partie de module de ventilateur (140) et de la partie de sortie (36) dans la direction de la largeur.

17. Appareil portable de traitement de l'air selon la revendication 15 ou 16, dans lequel :
une pluralité d'éléments de ventilateur (160) sont installés dans la direction verticale dans la partie de module de ventilateur (140) ; et
la partie d'assainissement (210) est installée sur une position de manière parallèle à une ligne virtuelle verticale (V) qui croise de manière perpendiculaire l'axe central de rotation des éléments de ventilateur (160) et s'étend dans la direction verticale, et/ou
une surface latérale de la partie d'assainissement (210) est supportée par une partie faisant saillie de fixation (116) du carter de filtre (80) ; et
l'autre surface latérale de la partie d'assainissement (210) est supportée par la partie de module de ventilateur (140).
